# EUROPEAN PATENT APPLICATION

(11) **EP 0 523 846 A2**
(43) Date of publication of application: **20.01.1993**
(21) Application number: 92305392.0
(22) Date of filing: 12.06.1992
(51) Int. Cl.: A61K 31/55

(54) **1,4-Benzodiazepines with 5-membered heterocyclic rings**

(30) Priority: 14.06.1991 US 715528
(71) Applicant: MERCK & CO. INC., Rahway New Jersey 07065-0900 (US)
(72) Inventor: Freidinger, Roger M., Lansdale, PA 19446 (US); Evans, Ben E., Lansdale, PA 19446 (US); Bock, Mark G., Hatfield, PA 19440 (US)
(74) Representative: Thompson, John Dr.

(57) **Abstract**

This invention relates to pharmaceutical compositions containing 1,4-benzodiazepines with fused 5-membered heterocyclic rings which are useful in the treatment of panic disorder or anxiety disorder.

## Description

### BACKGROUND OF THE INVENTION

Cholecystokinins (CCK) and gastrin are structurally-related neuropeptides which exist in gastrointestinal tissue and in the the central nervous system (see, V. Mutt, Gastrointestinal Hormones, G. B. J. Glass, Ed., Raven press, N.Y., p. 169 and G. Nisson, ibid, p. 127).

The isolation of the 33-amino acid polypeptide, cholecystokinin (CCK-33), from porcine intestine, Mutt, V. et al., "Structure of Porcine Cholecystokininpancreozymin. 1. Cleavage with Thrombin and Trypsin", European J. Biochem. 6, 156, (1968), was followed by the discovery that it occurs in numerous molecular forms at various sites throughout the peripheral and central nervous systems, Larsson, L. et al., "Localization and Molecular Heterogeneity of Cholecystokinin in the Central and peripheral Nervous System", Brain Res., 165, 201 (1979). In the mammalian brain the predominant fragments are the carboxy terminal octapeptide, H-Asp-Tyr(SO₃H)-Met-Gly-Trp-Met-Asp-Phe-NH₂ (CCK-8s, CCK₂₆₋₃₃) and tetrapeptide, CCK-4 (CCK₃₀₋₃₃).

The carboxy terminal octapeptide possesses the full biological profile of CCK, Dockray, G.J. et al., "Isolation, Structure and Biological Activity of Two Cholecystokinin Octapeptides from Sheep Brain", Nature 274, 711 (1978), and meets many anatomical and biochemical criteria which characterize a neurotransmitter, Vanderhaeghen, J.J. et al., "J. Neuronal Cholecystokinin", Ann. N,Y. Acad. Sci., 448, (1985). The presence of high concentrations of CCK-8s in the mammalian CNS is complemented with findings of specific and high affinity membrane-bound CCK binding sites, Innis, R.B. et al., "Distinct Cholecystokinin Receptors in Brain and pancreas", Proc. Natl. Acad. Sci. U.S.A., 77, 6917 (1980).

Evidence that more than one form of CCK receptor might exist was first provided in 1980 by Innis and Snyder, Innis, R.B. et al., "Distinct Cholecystokinin Receptors in Brain and Pancreas", Proc. Natl. Acad. Sci. U.S.A., 77, 6917 (1980) . At present, CCK receptors have been differentiated into primarily two subtypes based on their affinity for CCK fragments and analogues, Innis, R.B. et al., "Distinct Cholecystokinin Receptors in Brain and Pancreas", Proc. Natl. Acad. Sci. U.S.A., 77, 6917 (1980). The subsequent development of agents which discriminate between different CCK receptor types afforded further support for these assignments, Chang, R.S.L. et al., "Biochemical and pharmacological Characterization of an Extremely Potent and Selective Nonpeptide Cholecystokinin Antagonist", Proc. Natl. Acad. Sci. U.S.A., 83, 4923 (1986).

The CCK-A receptors, previously known as peripheral CCK receptors, are located in organs such as the pancreas, gallbladder, and colon. They exhibit high affinity for CCK-8s and a lower affinity for the corresponding desulphated fragment, CCK-8d, for CCK-4, and gastrin. Recent autoradiographic results have localized CCK-A receptors in the brain as well, Hill, D.R. et al., "Autoradiographic Localization and Biochemical Characterization of Peripheral Type CCK Receptors in Rat CNS Using Highly Selective Nonpeptide CCK Antagonists", J. Neurosci., 7, 2967 (1987).

The majority of the CCK receptors in the brain are of the CCK-B type. These were previously designated as central CCK receptors. CCK-B receptors are widely distributed throughout the brain and display high affinity for CCK-8s, CCK-4, and pentagastrin, Hill, D.R. et al., "Autoradiographic Localization and Biochemical Characterization of peripheral Type CCK Receptors in Rat CNS Using Highly Selective Nonpeptide CCK Antagonists", J. Neurosci, 7, 2967 (1987).

In addition to the above mentioned CCK receptor subtypes is a third type, the stomach gastrin receptor, which appears to be closely related to the CCK-B receptor subtype, Beinfeld, M.C., "Cholecystokinin in the Central Nervous System; a Minireview", Neuropeptides, 3, 4111 (1983). The minimum fully potent CCK sequence at this receptor is CCK-4, Gregory, R.A., "A Review of some Recent Development in the Chemistry of the Gastrins", Biorg. Chem., 8,497 (1979).

A wide range of physiological responses has been attributed to CCK. In an effort to elucidate its biological roles, researchers have relied primarily on a collection of CCK-A antagonists which has been steadily supplemented and improved to now include very selective, high-affinity agents, Evans, B.E., "Recent Developments in Cholecystokinin Antagonist Research," Drugs Future, 14, 971 (1989). In addition to their value as investigative tools, CCK antagonists retain considerable therapeutic potential, Gertz, B.J., "potential Clinical Applications, of a CCK Antagonist in Cholecystokinin Antagonists," Alan R. Liss, Inc.: New York, pp. 327 (1988).

In recent years, interest in agonists and antagonists of CCK has been stimulated by the possible clinical application of such compounds, Silverman, M.A. et al., "Cholecystokinin Receptor Antagonists, a Review", Am. J. Gastroenterol, 82, 703, (1987). The discovery of the presence of CCK in the brain and its significance in relation to its modulation of dopaminergic functions, effects on satiety, its roles in nociception, in anxiety, and other brain functions, Vanderhaeghen, J.J., et al., "J. Neuronal Cholecystokinin", Ann. N.Y. Acad. Sci. 448 (1985) has understandably intensified the search for CCK-B selective agents. Since the relevant biologically active fragment, CCK-8s, has a half-life of less than 1 hour, Deschodt-Lanckman, K., et al., "Degradation of Cholecystokinin-like Peptides by a Crude Rat Brain Synaptosomal Fraction: a Study by High Pressure Liquid Chromatography", Reg. Pept., 2, 15 (1981), implicit in the development of candidates for clinical use are criteria of high potency, selectivity, long in-vivo duration, oral bioavailability, and capability of penetrating the blood-brain barrier. These are strict prerequisites, given the tenuous stature of peptides as drugs, Veber, D.F., et al., "The Design of Metabolically-stable Peptide Analogs", Trends Neurosci. 8, 392 (1985).

Nevertheless, by employing stratagems which stabilize peptide structures, advances have been made toward developing highly potent and selective peptidal CCK-B receptor ligands Charpentier, B. et al., "Cyclic Cholecystokinin Analogues with High Selectivity for Central Receptors". Proc. Natl. Acad. Sci. U.S.A., 85, 1968, (1988). Analogues are now available which have proven resistant to enzymatic degradation Charpentier, B. et al., "Enzyme-resistant CCK Analogs with High Affinities for Central Receptors", Peptides, 9 835 (1988). Despite favorable receptor binding profiles, this class of compounds fails to meet previously cited key requirements which characterize a drug candidate. In response, researchers have turned to non-peptide compounds which offer a broader range of structure and physicochemical properties.

It was, therefore, an object of this invention to identify pharmaceutical compositions containing the compounds of Formula I which are useful in the treatment of panic disorder or anxiety disorder in a mammal, especially a human. The compounds of Formula I are also useful in the treatment of oncologic disorders, controlling pupil constriction in the eye, or preventing a withdrawal response produced by treatment or abuse of drugs or alcohol.

### SUMMARY OF THE INVENTION

The present invention is directed to pharmaceutical compositions containing 1,4-benzodiazepines with fused 5-membered heterocyclic rings which are useful in the treatment of panic disorder or anxiety disorder in a mammal, especially in a human. The compounds of Formula I are also useful in the treatment of oncologic disorders, controlling pupil constriction in the eye, or treating a withdrawal response produced by treatment or abuse of drugs or alcohol.

### DETAILED DESCRIPTION OF THE INVENTION

The pharmaceutical composition of this invention contain 1,4-benzodiazepines with fused 5-membered heterocyclic rings of Formula I: 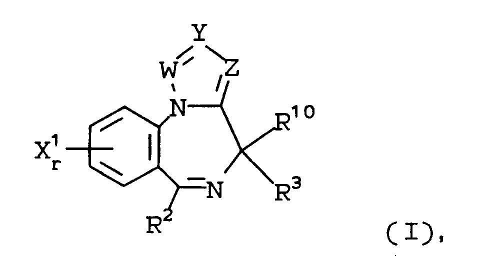
wherein
R¹ is H, C₁-C₄-straight- or branched-chain alkyl, cyclo-C₃-C₇-alkyl, or unsubstituted or mono- or disubstituted phenyl (where the substituent(s) is/are selected from the group consisting of halo, C₁-C₄-alkyl, C₁-C₄-alkoxy, and hydroxy);
R² is H, C₁-C₄-straight- or branched-chain alkyl, mono- or disubstituted or unsubstituted phenyl (where the substituent(s) is/are selected from the group consisting of halo, C₁-C₄-alkyl, C₁-C₄-alkoxy, C₁-C₄-alkylthio, carboxyl, carboxyl-C₁-C₄-alkyl, nitro, -CF₃, 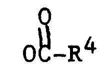
and hydroxy), 2-, 3- or 4-pyridyl or -(CH₂)ₘCOOR⁶;
R³ is 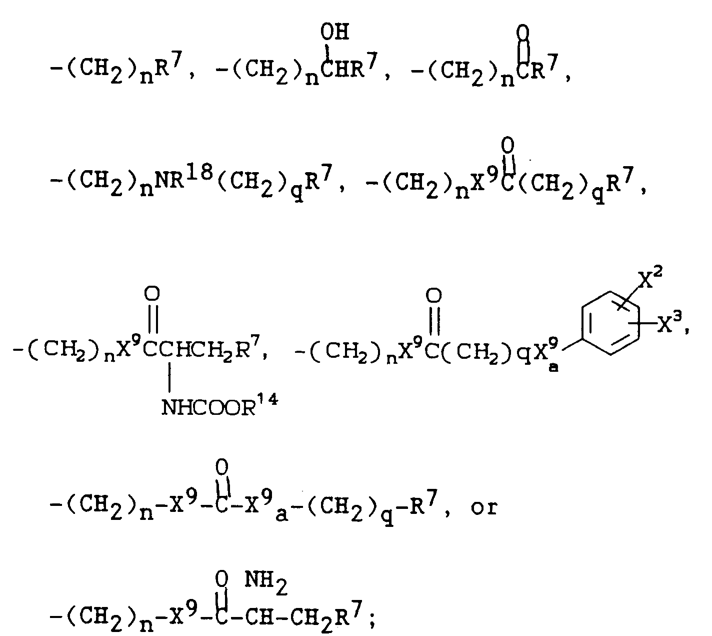
R⁴ and R⁵ are independently H, C₁-C₄-straight- or branched-chain-alkyl, cyclo-C₃-C₇-alkyl, or are connected to form a hetero ring of the form,-N(CH₂)ₖ, where k is 2 to 6;
R⁶ is H, C₁-C₄-straight or branched-chain alkyl, cyclo-C₃-C₅-alkyl, unsubstituted or mono- or disubstituted phenyl (where the substituent(s) is/are selected from the group consisting of halo, C₁-C₄-alkyl, C₁-C₄-alkoxy, nitro, and CF₃), or unsubstituted or mono- or disubstituted phenyl-C₁-C₄-straight or branched-chain alkyl (where the substituent(s) is/are selected from the group consisting of halo, C₁-C₄-alkyl, C₁-C₄-alkoxy, nitro, and CF₃);
R⁷ is a- or β-naphthyl, unsubstituted or mono- or disubstituted phenyl (where the substituent(s) is/are selected from the group consisting of halo, -NO₂, -OH, -NR⁴R⁵, C₁-C₄-straight- or branched-chain alkyl, cyano, phenyl, trifluoromethyl, acetylamino, acetyloxy, C₁-C₄-straight- or branched-chain alkylthio, SCF₃, C=CH, CH₂SCF₃, OCHF₂, S-phenyl, or C₁-C₄-straight- or branched-chain alkoxy), 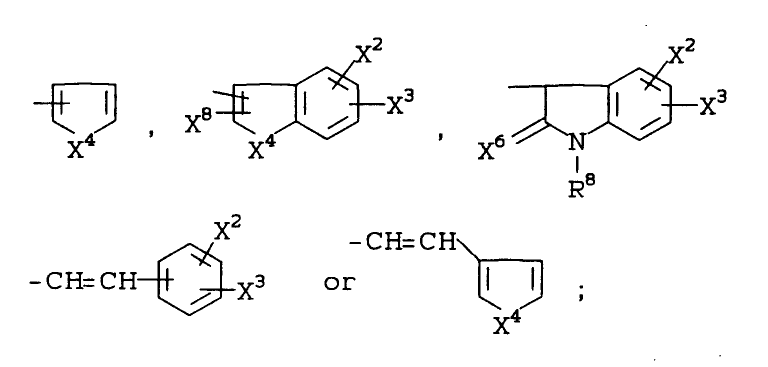
R⁸ is H, C₁-C₄-straight- or branched-chain alkyl, cyclo-C₃-C₇-alkyl, -(CH₂)ₙ-cyclo-C₃-C₇-alkyl, 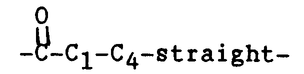
or branched-chain alkyl, or 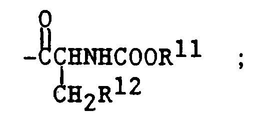
R⁹ is OH, OR¹¹ or 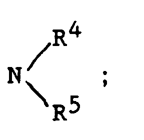
R¹⁰ is H, -OH, or -CH₃;
R¹¹ and R¹² are independently C₁-C₄-straight- or branched-chain alkyl or cyclo-C₃-C₇-alkyl;
R¹³ is H or C₁-C₄-straight- or branched-chain alkyl, cyclo-C₃-C₇-alkyl, 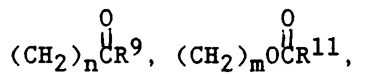
(CH₂)ₘNHR¹⁵, (CH₂)ₘOH, or phenyl-C₁-C₄-straight- or branched-chain alkyl;
R¹⁴ is C₁-C₄-straight- or branched-chain alkyl or phenyl-C₁-C₄-straight- or branched-chain alkyl;
R¹⁵ is H or 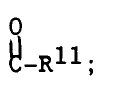
R¹⁶ is H, C₁-C₄-straight- or branched-chain alkyl, cyclo-C₃-C₇-alkyl, or phenyl-C₁-C₄-straight- or branched-chain alkyl;
R¹⁷ is H, C₁-C₄-straight- or branched-chain alkyl, COOR¹⁶, phenyl or phenyl-C₁-C₄-straight- or branched-chain alkyl;
R¹⁸ is H, C₁-C₄-straight- or branched-chain alkyl or formyl, acetyl, propionyl or butyryl;
m is 1-to-4;
n is 0-to-4;
q is 0-to-4:
r is 1 or 2;
X¹ is H, -NO₂, CF₃, CN, OH, C₁-C₄-straight- or branched-chain alkyl, halo, C₁-C₄-straight- or branched-chain alkylthio, C₁-C₄-straight- or branched-chain alkoxy, -(CH₂)ₙCOOR⁶, -NR⁴R⁵, or 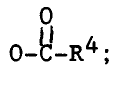
X² and X³ are independently H, -OH,-NO₂, halo, C₁-C₄-straight- or branched-chain alkylthio, C₁-C₄-straight- or branched-chain alkyl, C₁-C₄-straight- or branched-chain alkoxy, or 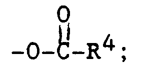
X⁴ is S, O, CH₂, or NR⁸;
X⁶ is O or HH;
X⁸ is H or C₁-C₄-straight- or branched-chain alkyl;
X⁹ and X⁹ₐ are independently NR¹⁸ or O;
W = CR¹ or N;
Y = CR¹³ or N; or
Z = N or CR¹⁷;
or a pharmaceutically acceptable salt thereof and a pharmaceutically acceptable carrier.

As used herein, the definition of each expression, i.e., m, n, p, C₁-C₄-alkyl, etc., when it occurs more than once in any structure, is intended to be independent of its definition elsewhere in the same structure.

In the pharmaceutical compostions containing the compounds of Formula I, the preferred stereochemistry for CCK-antagonism relates to D-tryptophan, where C^{3a} and N⁵ of Formula I correspond to the carbonyl carbon and a-amino nitrogen of D-tryptophan and R³ occupies the position of the indolylmethyl side chain.

In the pharmaceutical compositions containing the compounds of Formula I, the preferred stereochemistry for CCK-B and gastrin antagonism may be either D or L depending on the nature of R³. For example, when R³ = (CH₂)ₙR⁷ or 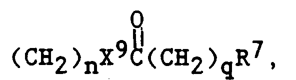
the preferred stereochemistry corresponds to D-tryptophan, as above. When 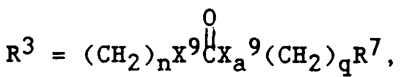
the preferred stereochemistry corresponds to L-tryptophan.

As used herein, halo is F, Cl, Br, or I; and C₁-C₄-alkyl includes methyl, ethyl, propyl, isopropyl, butyl, isobutyl, and t-butyl.

Preferred pharmacetuical compositions containing the compounds according to the instant invention are those wherein R¹ is H or C₁-C₄-alkyl; R² is unsubstituted or mono- or disubstituted phenyl (wherein the substituents are selected from the group consisting of halo, C₁-C₄-alkyl, C₁-C₄-alkoxy, nitro and -CF₃); R³ is -(CH₂)ₙR⁷, 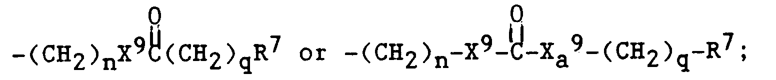
R⁴ and R⁵ are independently H, C₁-C₄-alkyl or are connected to form the ring -N(CH₂)ₖ, where k is 4or 5; R⁷ is a- or β-naphthyl, unsubstituted or mono- or disubstituted phenyl (wherein the substituents are selected from the group consisting of halo, -NO₂, C₁-C₄-alkyl, C₁-C₄-alkoxy and trifluoromethyl), 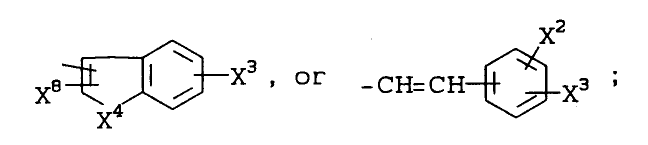
R⁸ is H or C₁-C₄-alkyl; R¹¹ is C₁-C₄-alkyl; R¹³ is H, C₁-C₄-alkyl, cyclo-C₃-C₇-alkyl or 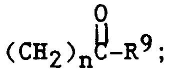
R¹⁶ is C₁-C₄-alkyl; n is 0-to-2; q is 0-to-2; X¹ is H, -NO₂, C₁-C₄-alkyl, halo or C₁-C₄-alkoxy; X² and X³ are independently H, -NO₂, halo, C₁-C₄-alkyl or C₁-C₄-alkoxy; and X⁹ and Xₐ⁹ are independently NH or O.

Particularly preferred pharmaceutical conpositions containing the compounds include those wherein R¹ is H or methyl; R² is phenyl or o-F-phenyl; R³ is 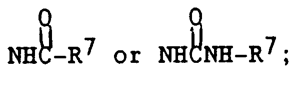
R⁷ is 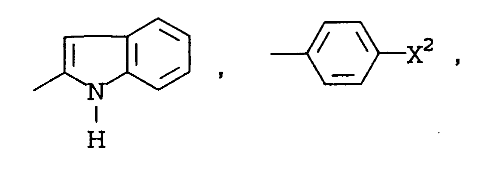 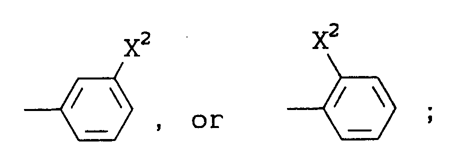
R¹³ is H, methyl, ethyl or propyl; R¹⁷ is H, methyl or ethyl: X¹ is H: X² is H, -NO₂, halo, methyl or methoxy; W is CR¹; Y is CR¹³ and Z is CR¹⁷. Other particularly preferred compounds include those wherein R¹ is H or methyl: R² is phenyl or o-F-phenyl; R³ is 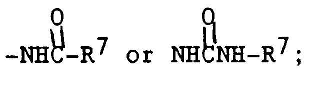
R⁷ is 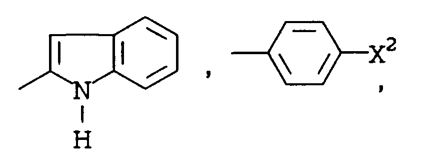 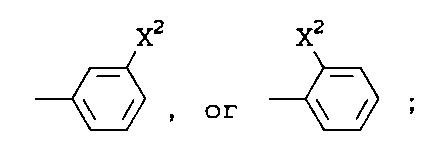
R¹³ is H; X¹ is H; X² is H, -NO₂, halo, methyl or methoxy; W is CR¹; Y is CR¹³; and Z is N. Still other particularly preferred compounds include those wherein R¹ is H or methyl; R² is phenyl or o-F-phenyl; R³ is 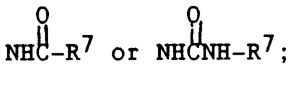
R⁷ is 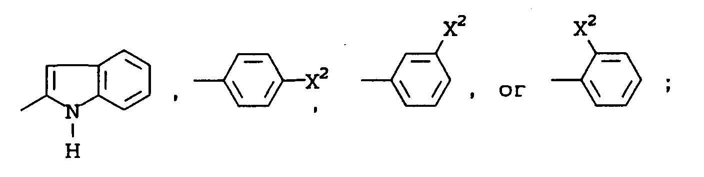
R¹⁷ is H, CH₃, CH₂CH₃, COOH, COOCH₃, or COOCH₂CH₃; X¹ is H; X² is H, -NO₂, halo, methyl, or methoxy; W is CR¹; Y is N and Z is CR¹⁷. Yet other particularly preferred compounds include those wherein R² is phenyl or o-F-phenyl; R³ is NHC-R⁷ or NHCNH-R⁷; R⁴ and R⁵ are independently H, methyl, ethyl or -(CH₂)₄-; R⁷ is 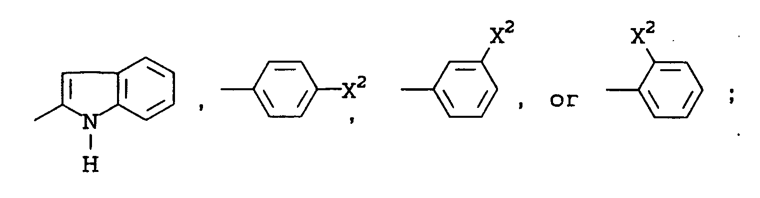
R⁹ is OH, OCH₃, OCH₂CH₃, or 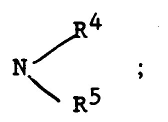
R¹³ is methyl, ethyl, propyl or 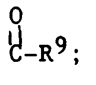
R¹⁷ is H; X¹ is H; X² is H, -NO₂, halo, methyl, or methoxy; W is N; Y is CR¹³; and Z is CR¹⁷. Then, other particularly preferred compounds include those wherein R² is phenyl or o-F-phenyl; R³ is 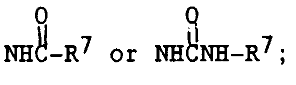
R⁷ is 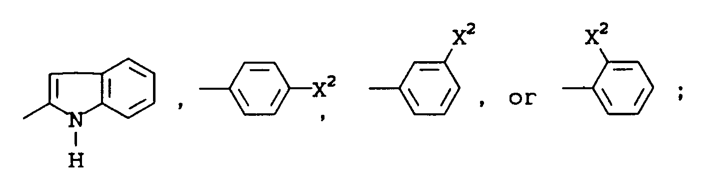
R¹⁷ is H, methyl, COOCH₃, or COOCH₂CH₃; X¹ is H; X² is H, -NO₂, halo, methyl or methoxy; W is N; Y is N; Z is CR¹⁷. Still other particularly preferred pharmaceutical compostions containing the compounds of this invention include those wherein R² is phenyl or o-F-phenyl; R³ is 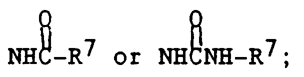
R⁷ is 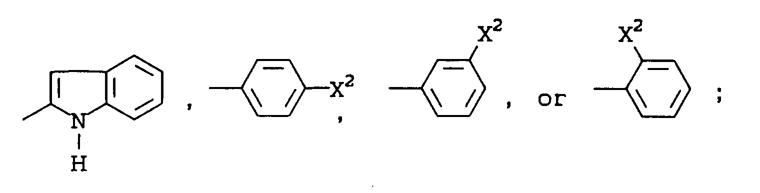
X¹ is H, X² is H, -NO₂, halo, methyl or methoxy; W is N; Y is N; and Z is N.

Even more particularly preferred pharmaceutical composition containing the compounds of this invention include, for CCK antagonism:
4(S)-4(2-indolecarbonylamino)-6-phenyl-4H-pyrrolo[1,2-a]-1,4-benzodiazepine;
4(S)-4(4-chlorophenylcarbonylamino)-6-phenyl-4H-imidazo-[1,2-a]-1,4-benzodiazepine;
4(S)-4-(2-indolecarbonylamino)-1-methyl-6-phenyl-3-propyl-4H-imidazo-[1,5-a]-1,4-benzodiazepine;
4(S)-4-(2-indolecarbonylamino)-2-methyl-6-phenyl-4H-pyrazolo-[1,5-a]-1,4-benzodiazepine;
4(S)-4-(2-indolecarbonylamino)-3-methyl-6-phenyl-4H-triazolo-[1,5-a]-1,4-benzodiazepine; and
4(S)-4-(2-indolecarbonylamino)-6-phenyl-4H-tetrazolo-[1,5-a]-1,4-benzodiazepine;
and for CCK-B and gastrin antagonism
4(R)-4(3-methoxyphenylaminocarbonylamino)-6-phenyl-4H-pyrrolo-[1,2-a]-1,4-benzodiazepine;
4(R)-4-(3-methylphenylaminocarbonylamino)-6-phenyl-4H-imidazo-[1,2,-a]-1,4-benzodiazepine;
4(R)-4-(3-chlorophenylaminocarbonylamino)-1-methyl-6-phenyl-3-propyl-4H-imidazo-[1,2-a]-1,4-benzodiazepine;
4(R)-4-(3-methoxyphenylaminocarbonylamino)-6-phenyl-4H-pyrazolo-[1,5-a]-1,4-benzodiazepine-2-carboxylic acid, ethyl ester;
4(R)-4-(3-methylphenylaminocarbonylamino)-3-methyl-6-phenyl-4H-triazolo-[1,5-a]-1,4-benzodiazepine; and
4(R)-4-(3-chlorophenylaminocarbonylamino)-6-phenyl-4H-tetrazolo-[1,5-a]-1,4-benzodiazepine.

The pharmaceutically-acceptable salts of the compounds of Formula I include the conventional non-toxic salts or the quarternary ammonium salts of the compounds of Formula I formed, e.g., from non-toxic inorganic or organic acids. For example, such conventional non-toxic salts include those derived from inorganic acids such as hydrochloric, hydrobromic, sulfuric, phosphoric, nitric and the like; and the salts prepared from organic acids such as acetic, propionic, succinic, glycolic, stearic, lactic, malic, tartaric, citric, pamoic, maleic, hydroxymaleic, phenylacetic, glutamic, benzoic, salicylic, sulfanilic, 2-acetoxy-benzoic, fumaric, toluenesulfonic, methanesulfonic, ethane disulfonic, isethionic, and the like.

The pharmaceutical compositions containing the compounds of Formula I are particularly distinguished from benzodiazepines of the prior art by the presence of 3-substituents. These Formula I compounds bind strongly to CCK-receptors, but only weakly to benzodiazepine-receptors, especially with the increasing size of the 3-substituent.

The pharmaceutical compostions containing the compounds according to Formula I may be prepared according to Schemes I through XI as follows:

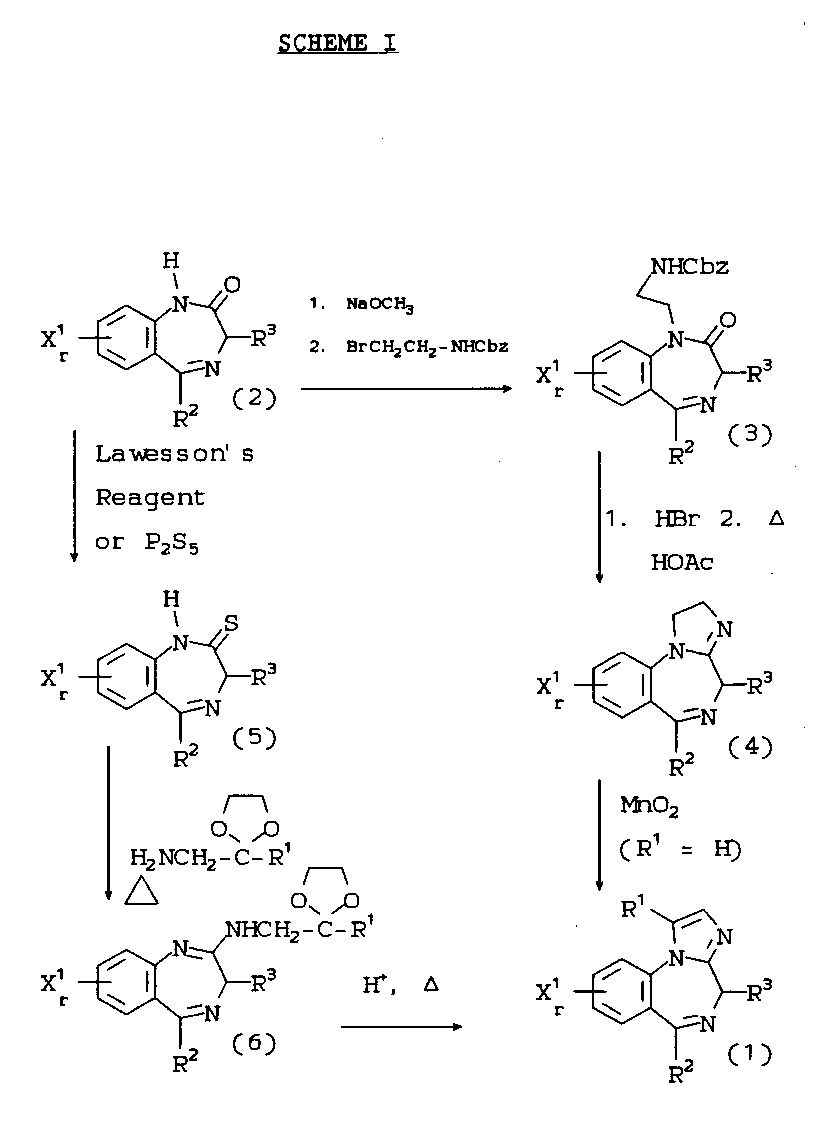

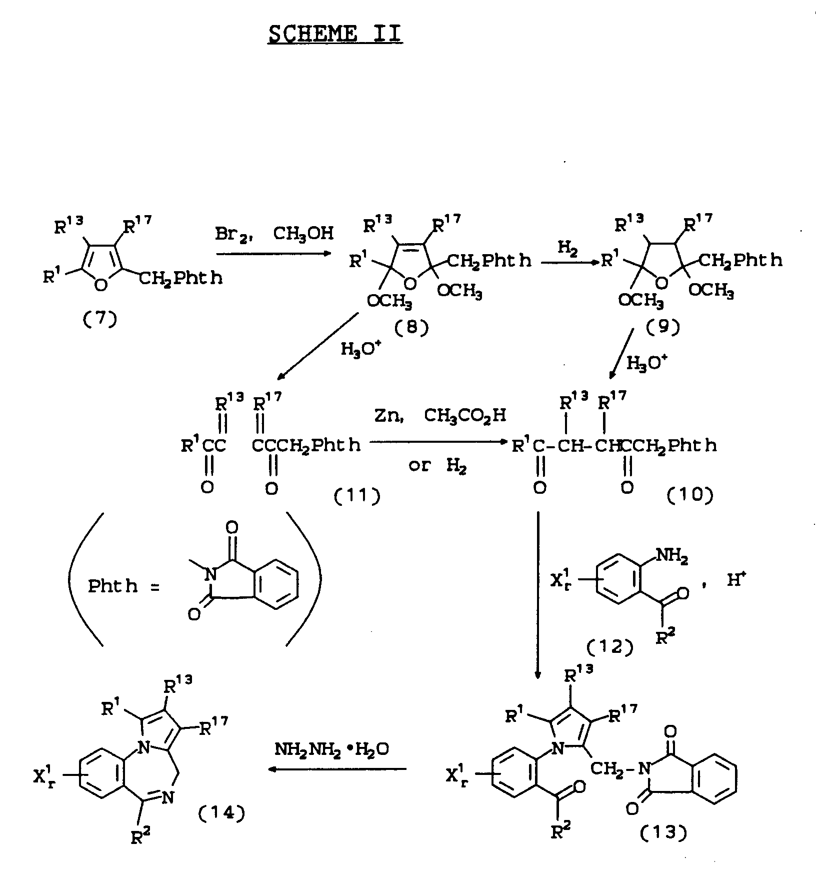

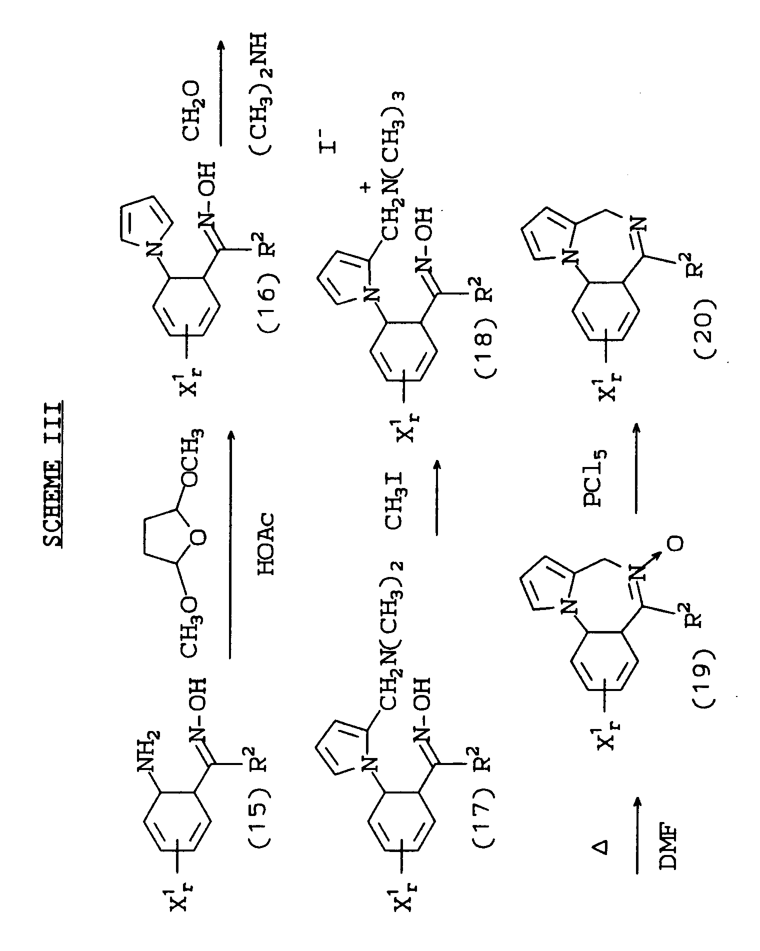

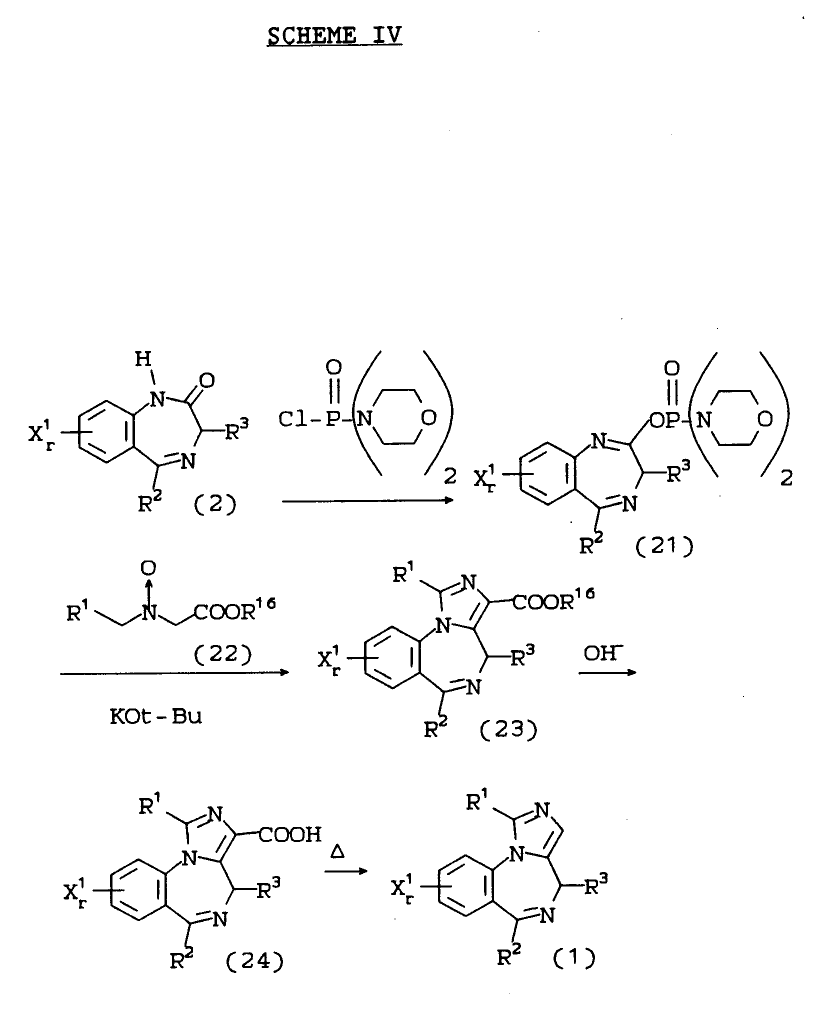

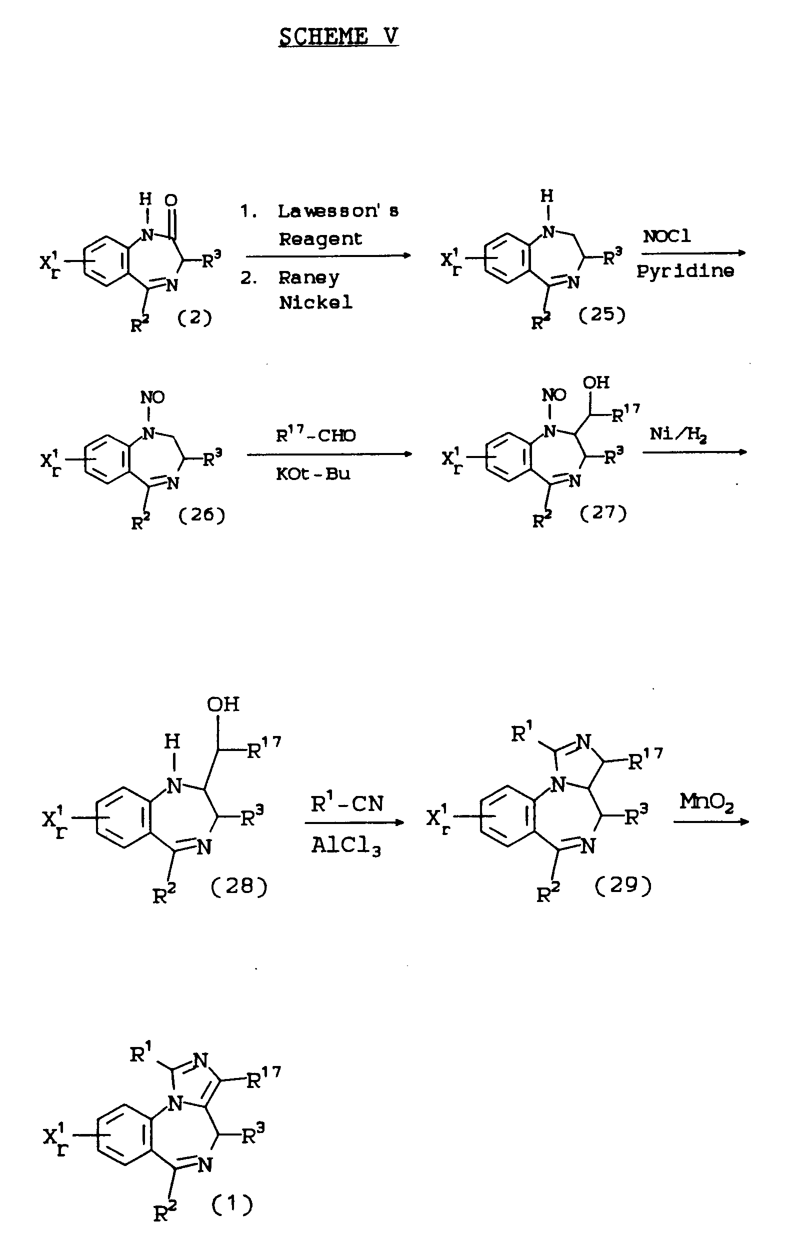

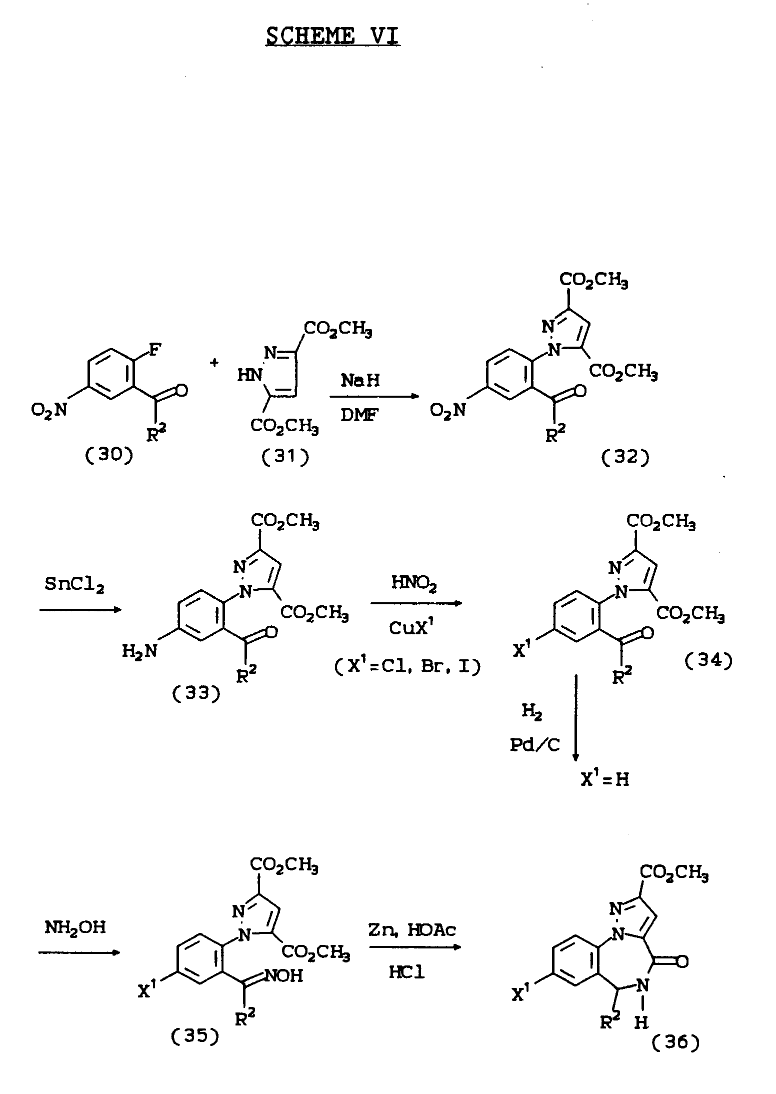

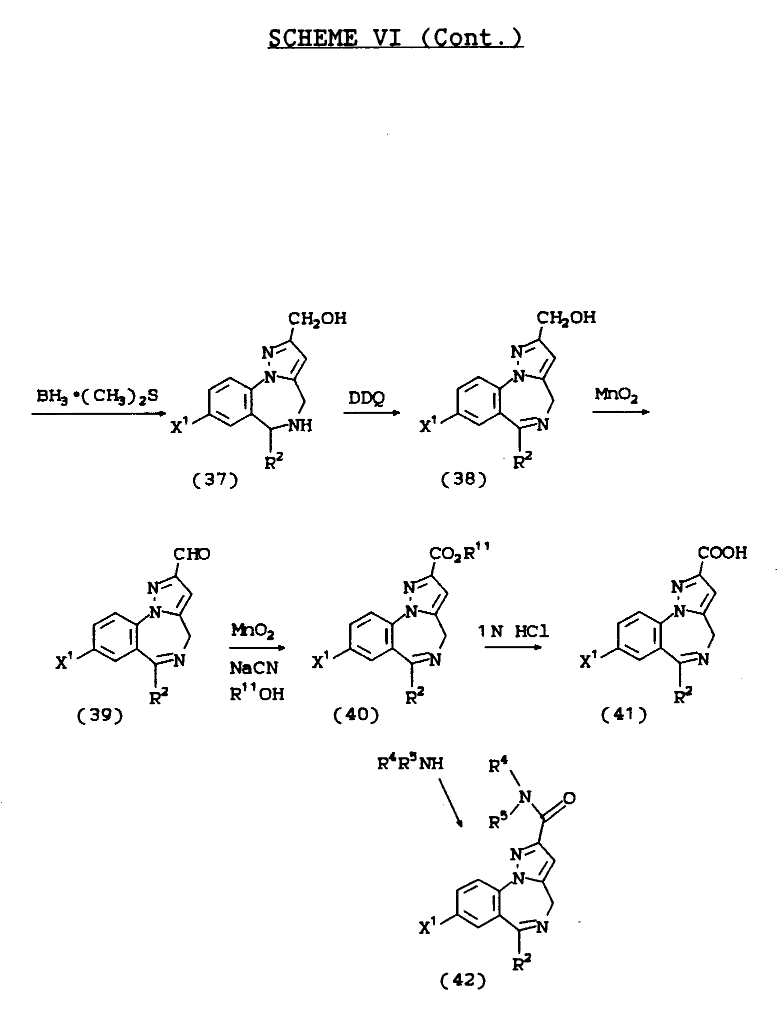

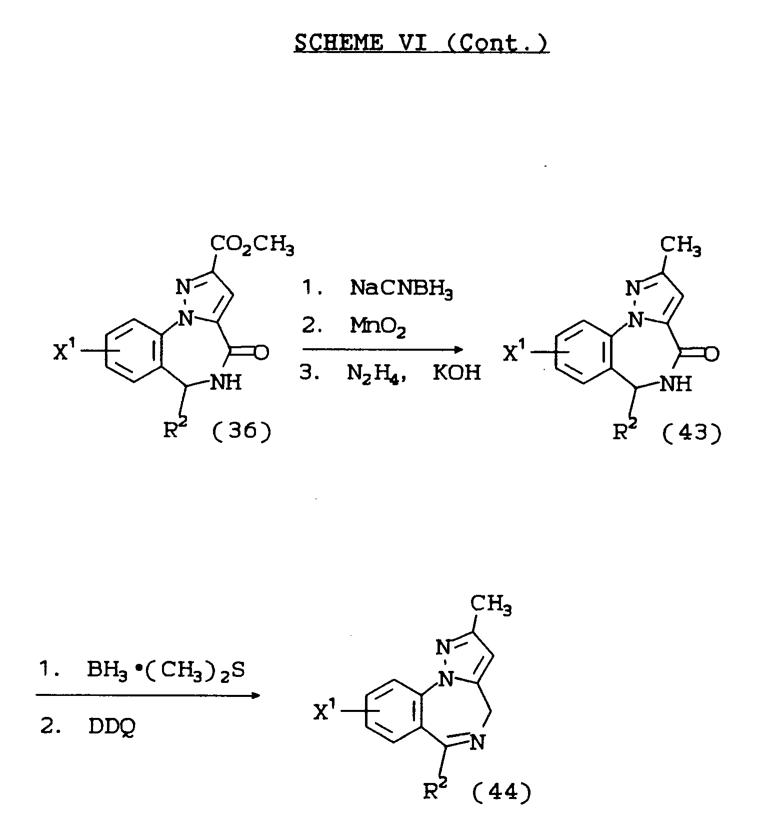

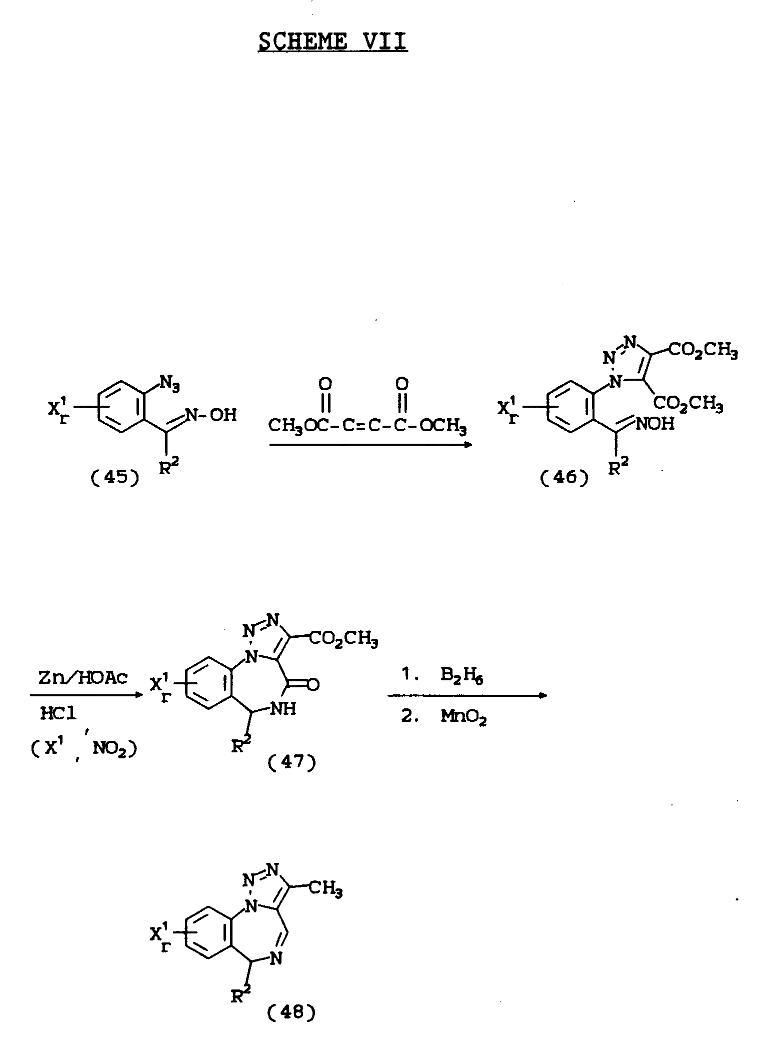

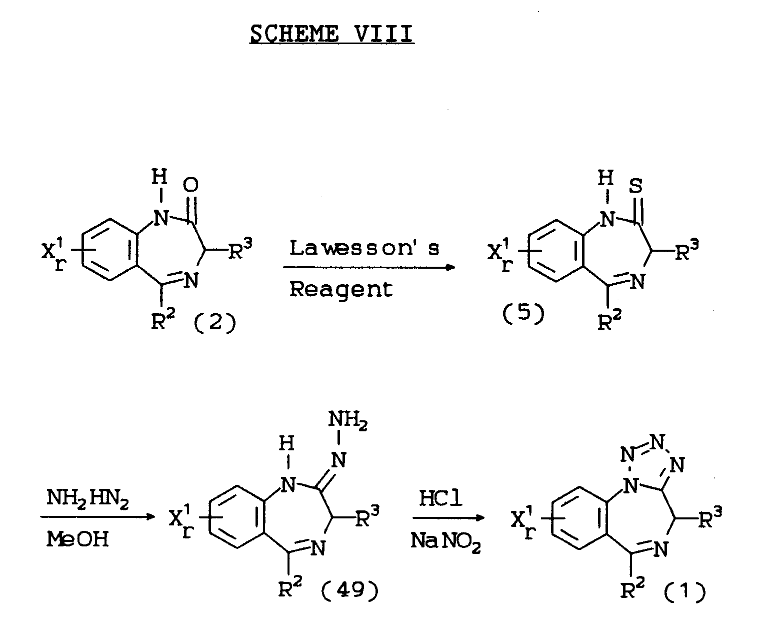

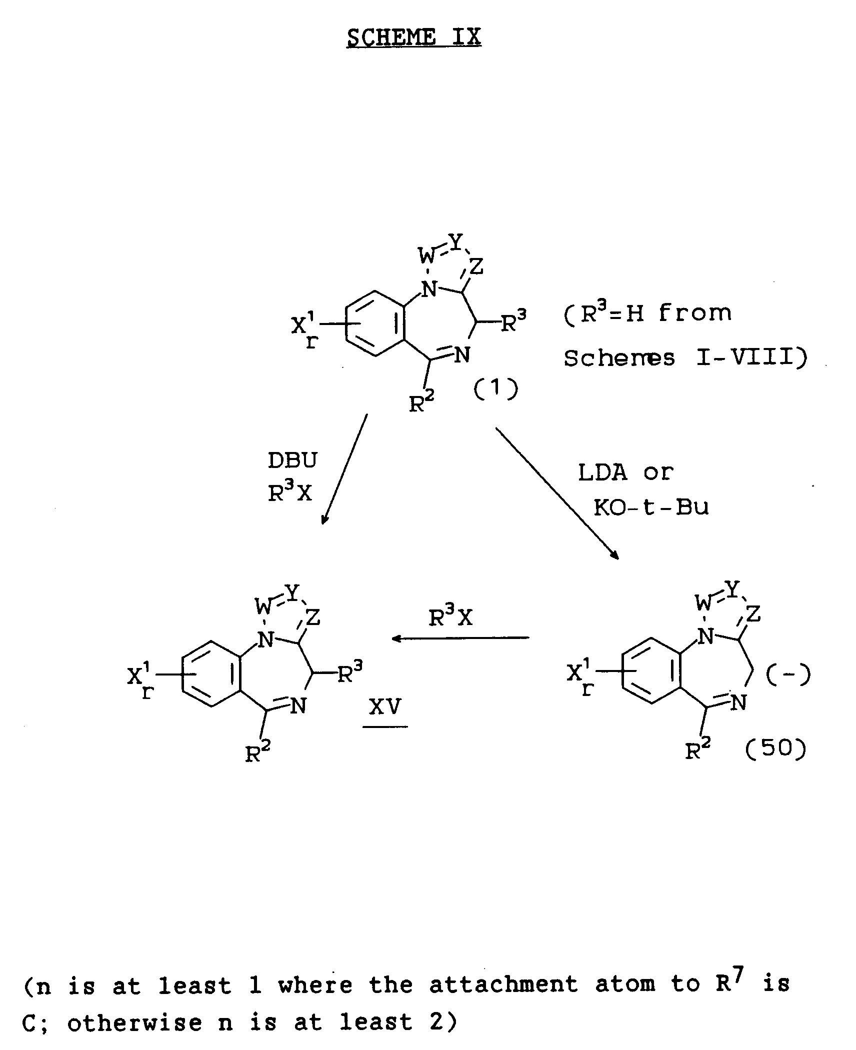

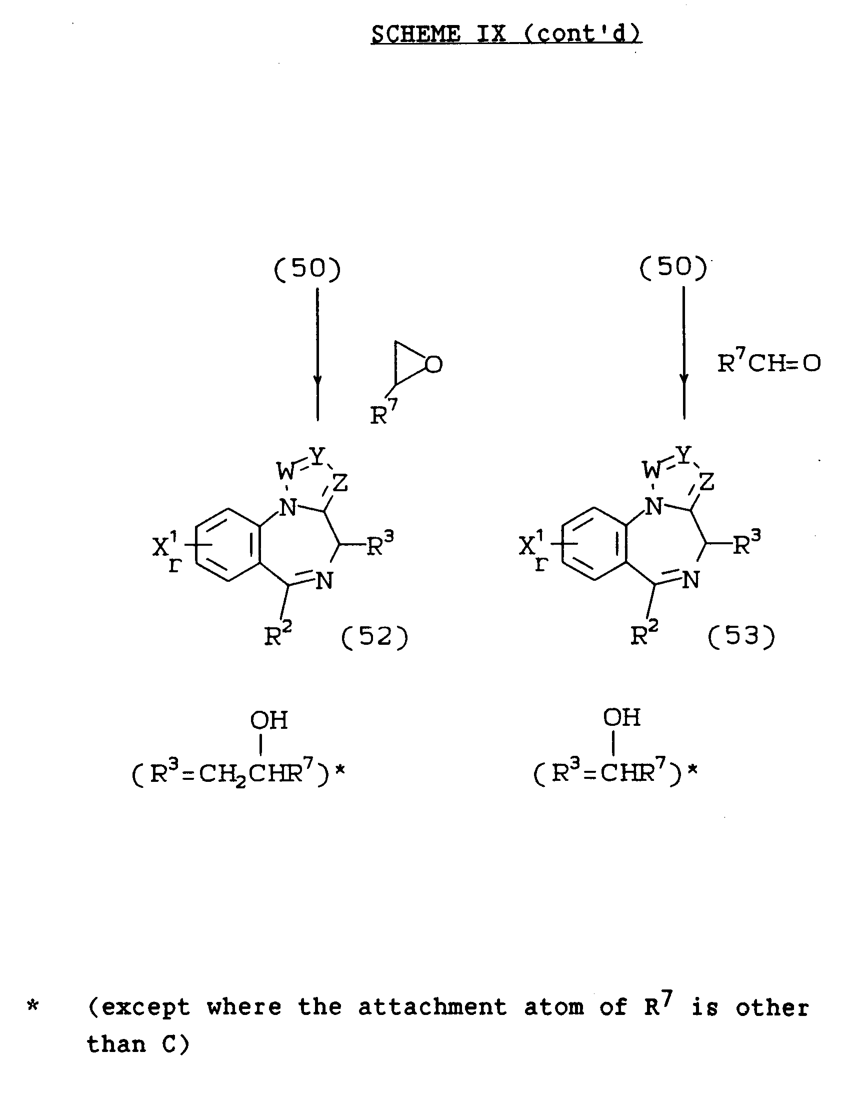

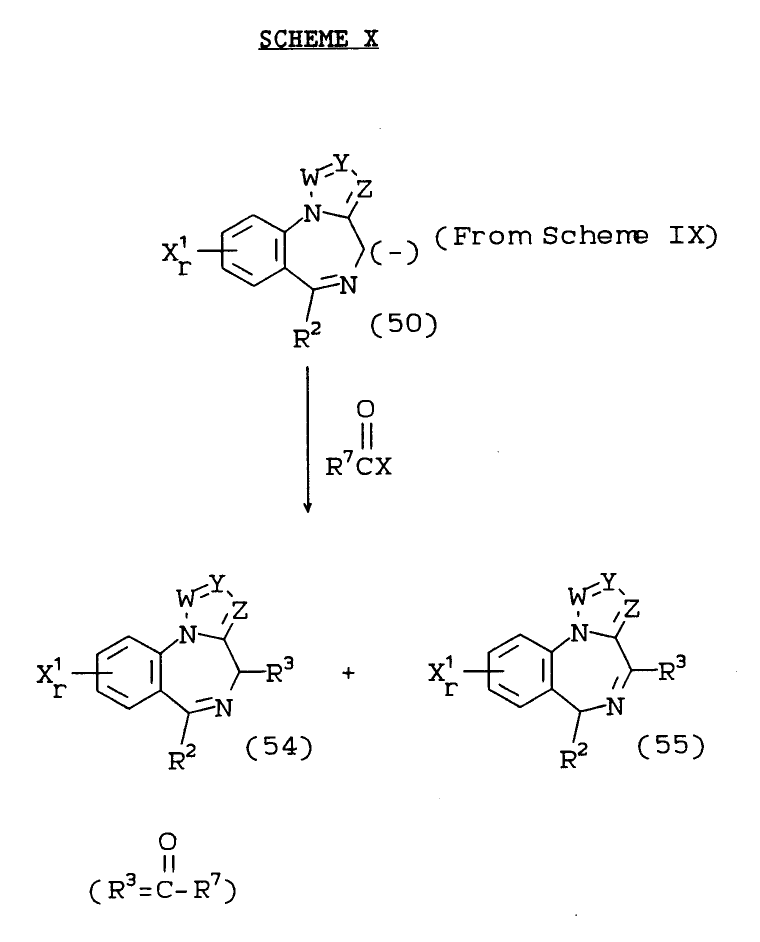

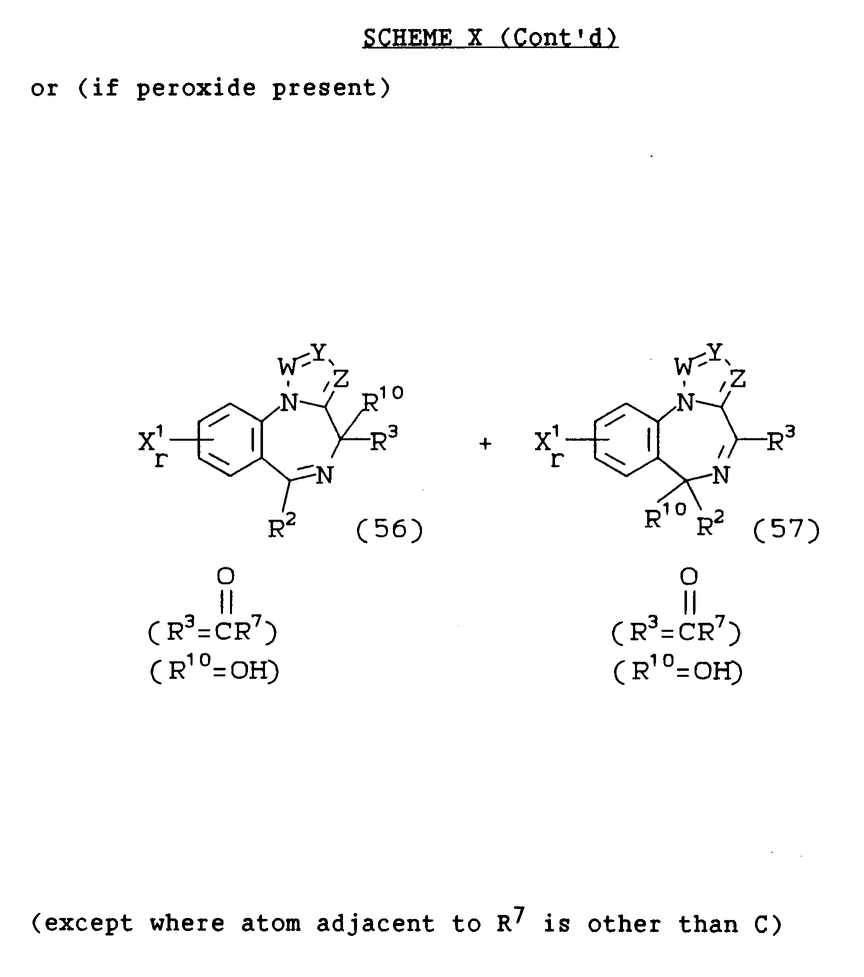

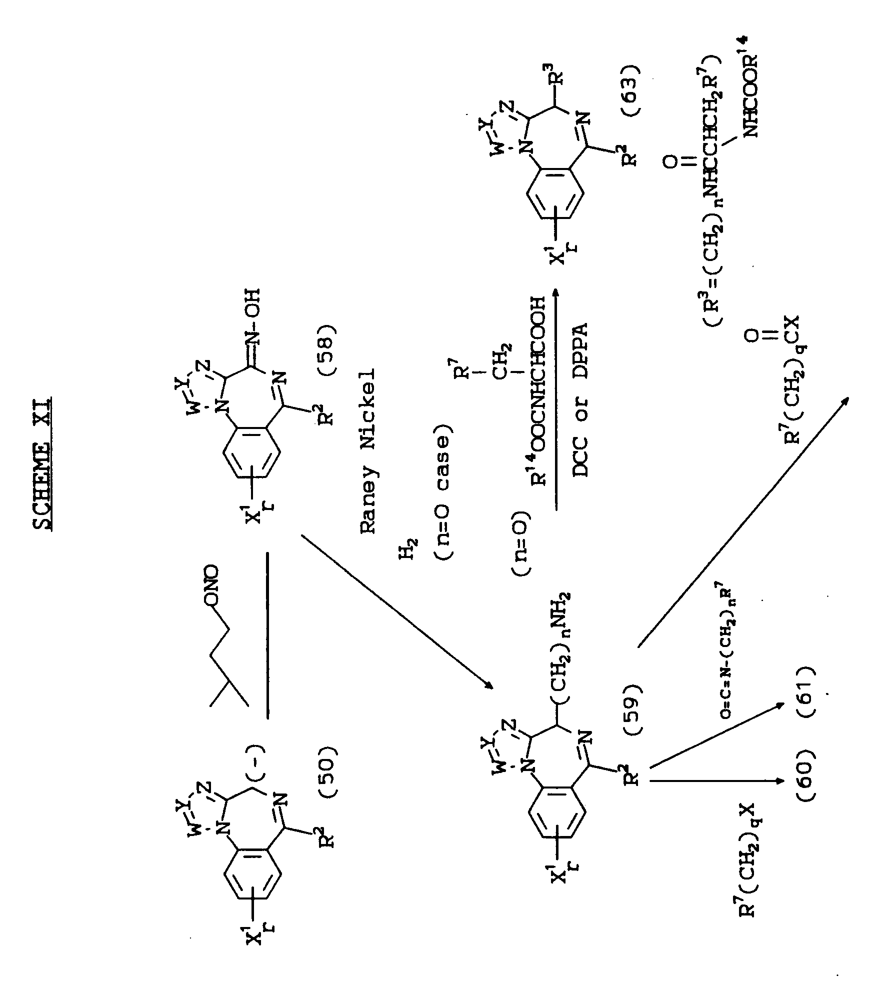

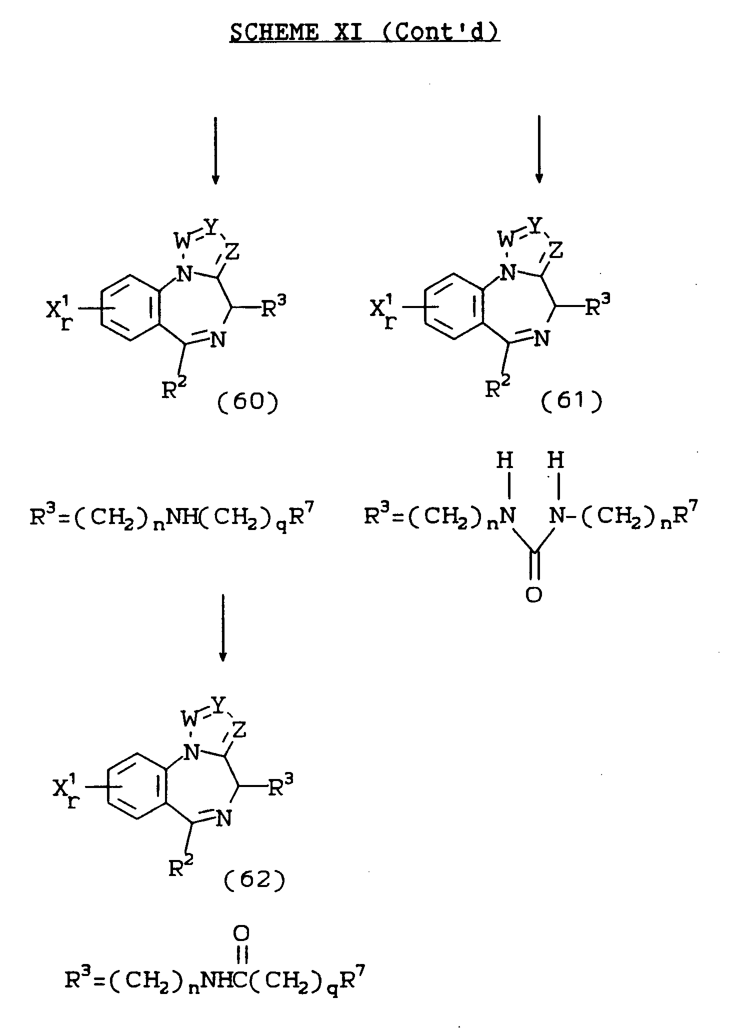

Referring to Reaction Scheme I, the benzodiazepines of Formula (2) which are readily available are used as the starting materials and are treated first with sodium methoxide and then with arbobenzoxybromoethylamine to give (3). Compound (3) is treated with a solution of HBr in glacial acetic acid to give the free amino derivative, and its dehydration product (4) formed by heating the amine under reflux in ethanol. Oxidation of (4) with manganese dioxide then produces the imidazobenzodiazepine of Formula (I). These latter compounds are also prepared by converting the . compound of Formula (2) into its thioamide (5) with Lawesson's reagent. Treatment with primary amine derivatives and heating in ethanol gives (6), which upon exposure to concentrated sulfuric acid gives the imidazobenzodiazepines (1). Alternate syntheses of Formula (I) compounds use the compound of Formula (I) (R³=H) and proceed according to Schemes IX, X, and XI.

Referring to Reaction Scheme II, treatment of 5-phthalimidomethylfuran (7) with bromine in a mixture of methylene chloride and absolute methanol at -40 to -50°C followed by introduction of gaseous ammonia gives the dihydrofuran (8) in an approximately equimolar mixture of trans and cis isomers.

Catalytic hydrogenation of (8) with the higher melting point using Raney nickel in ethanol affords the tetrahydrofuran (9). Hydrolysis of (9) gives ring-opened diketone (10). Alternatively, (10) may be prepared from (8) by hydrolysis to (11) followed by reduction with either zinc in acetic acid or by hydrogenation. Condensation of 2-aminobenzophenone (12) with (9) in benzene with p-toluene-sulfonic acid as catalyst gives the pyrrolylketone (13).

Removal of the phthaloyl group from (13) and ring closure to 1-methyl-6-phenyl-4H-pyrrolo[1,2-a]-[1,4]benzodiazepine (14) is effected by heating at reflux a solution of (13) in ethanol with hydrazine hydrate. Most of the phthalazine generated in the reaction may be removed as solid by filtration from the reaction mixture. Chromatographic isolation followed by recrystallization gives pyrrolobenzodiazepine (14). Compound (14) may then be converted to compounds of Formula I according to Schemes IX-XI.

An alternate route to pyrrolobenzodiazepines is outlined in Scheme III. Using the method of Clauson-Kaas for the synthesis of 1-substituted pyrroles, aminobenzophenone oximes (15) are treated with 2,5-dimethoxytetrahydrofuran to give the corresponding 2-(1-pyrrolyl)ketone derivatives (16). Treatment of the oximes with formaldehyde and dimethylamine then gives the Mannich bases (17), which are alkylated with methyl iodide to afford the corresponding quaternary salts (18). Cyclization to N-oxides (19) is achieved by heating in DMF and deoxygenation with pCl₅ gives pyrrolobenzodiazepines (20).

Compound (20) is converted to pyrrolobenzodiazepine compounds of Formula I according to Schemes IX-XI.

Referring to Reaction Scheme IV, imidazobenzodiazepines of Formula I are prepared using benzodiazepines of Formula (2) as starting materials. The Formula (2) compound is converted to iminophosphate (21) with dimorpholino phosphorochloridate. Condensation of the iminophosphate with the anion of the known nitrone, Compound 22, gives the imidazobenzodiazepine (23) in one step. Hydrolysis and decarboxylation gives (1) via (24).

A different approach is employed for the synthesis of the 1,3-substituted derivatives (1) (Scheme V). Compound (25) is synthesized from the Formula (2) benzodiazepine by conversion to the thioamide with Lawesson's reagent and desulfurization with Raney nickel. The 1-nitrosobenzodiazepine (26), which is readily obtained by reaction of compound (25) with nitrosyl chloride in pyridine, is condensed with aldehydes in the presence of potassium t-butoxide to give the diastereomeric alcohols (27). The diastereomers may be separated by chromatography if needed. Compounds (27) may then be reduced catalytically with Raney nickel to yield the corresponding amino alcohols (28).

Condensation of the amino alcohols (28) with acetonitrile and aluminum chloride gives the imidazoline (29) which need not be characterized, but is directly oxidized with activated manganese dioxide to yield the imidazole (1).

Certain compounds of Formula I are also prepared from imidazobenzodiazepines (1) (R³=H) according to the methods of Schemes IX-XI.

Referring to Reaction Scheme VI, the treatment of ketone (30) with the sodium salt of the pyrazolo diester, (31), in dimethylformamide leads to the ketone (32). The conversion of the nitro group to a halo substituent is carried out by a Sandmeyer procedure involving reduction of (32) with stannous chloride to give the amino compound (33). Diazotization with nitrous acid followed by treatment with cuprous halide gives the halo compound (34). Deshalo (34) is then obtained by hydrogenolysis with palladium on carbon. The synthesis of the pyrazolo[1,5-a][1,4]benzodiazepine ring system is achieved by the oximation of (34) to compound (35) which is reductively cyclized to (36) by treatment with zinc in acetic acid.

The reduction of the lactam ester, (36), to the dihydrobenzodiazepine, (37), is carried out with excess boranedimethylsulfide complex which reduces both the lactam and ester groups. The use of diborane in tetrahydrofuran gives a mixture of (37) in addition to the partially-reduced amino-ester. The oxidation of (37) with 2,3-dichloro-5,6-dicyano-1,4-benzoquinone (DDQ) yielded 8-halo[1,5-a][1,4]benzodiazepine (38).

The alcohol (38) is oxidized to the aldehyde (39) with manganese dioxide, rather than oxidizing the dihydroalcohol (37) directly to (39), which would result in lower overall yields.

The aldehyde, (39), is a versatile intermediate for the preparation of various 2-substituted pyrazolobenzodiazepines. The carboxylic ester, (40), is prepared from (39) by treatment with manganese dioxide in an alcohol in the presence of sodium cyanide. The ester is subsequently converted either to the acid, (41), by acid hydrolysis or to the amides, (42) by treatment with amines such as ammonia, methylamine and dimethylamine.

An additional pyrrazolobenzodiazepine, the 2-methyl analog, is prepared starting with compound (36). The ester function is reduced with sodium cyano borohydride to the alcohol, with oxidation with manganese dioxide giving the aldehyde, which in turn, under Wolff-Kishner conditions, gives the methyl compound (43). The product (44) is obtained by reduction of the amide group of (43) followed by DDQ oxidation.

Compound (44) is converted to the compounds of Formula I according to the methods of Schemes IX-XI.

Referring to Reaction Scheme VII, azido compound (45) undergoes a 1,3-dipolar cycloaddition with dimethylacetylene dicarboxylate to yield compound (46). The oxime of (46) is reduced with zinc in acetic acid which results in cyclization to amide (47). Reduction of the amide with diborane followed by oxidation with manganese dioxide produces (48) in which the ester is reduced to methyl. Compounds (48) are converted to Formula I compounds according to Schemes IX-XI.

Referring to Reaction Scheme VIII, thioamides (5) are converted to amidrazones (49) with hydrazine in methanol. Diazotization then produces tetrazolobenzodiazepines (1), with compounds (1) (R³=H) being converted to other compounds of Formula I according to Scheme IX-XI.

Referring now to Reaction Scheme IX, the anion (50) is generated from (1) by the procedure of J. Org. Chem., 46, 3945 (1981) using lithium diisopropylamide (LDA) or using potassium tert-butoxide.

The hydroxy alkyl derivative (53) is generated by adding an aldehyde to a solution of (50). Treatment of (50) with an epoxide yields the hydroxyethyl derivative (52) and by treating (50) with an alkyl halide, the alkyl derivative (51) is produced.

An alternative procedure for obtaining (51) is to treat (1) with an alkyl halide and a strong base such as 1,8-diazabicyclo[5.4.0]undec-7-ene (DBU) and heating.

These procedures also produce isomers of (51)-(53) which are analogous to (55) (Reaction Scheme X). Likewise, in the presence of peroxide, the analogs of the isomers and hydroxy derivatives (56) and (57) are produced.

Reaction Scheme X describes the formation of R³=keto compounds of Formula I. These are produced by treating the anion (50) with an acid halide or anhydride, with this reaction producing both isomers (54) and (55). When the reaction is run in the presence of peroxide, the hydroxy compounds (56) and (57) are produced.

Reaction Scheme XI describes the formation of Formula I compounds where R³ is a substituted amino or aminomethyl.

The heterocycle-fused benzodiazepines (59) are either known or readily derivable from known compounds. The amino compounds may also be obtained by nitrosation of (50) followed by reduction of the oxime (58) with Raney nickel and hydrogen.

When (59) is treated with an alkyl halide, the N-alkyl derivative (60) is produced.

When (59) is treated with an alpha-halo carboxylic acid derivative such as an a-halo acid, ester, amide, or the like, one obtains the corresponding a-amino compound (61).

Treatment of (59) with an acid halide or anhydride produces the N-acyl derivative (62).

Compound (59) may also be treated with an N-protected a-amino acid and a coupling reagent such as DCC or DPPA (diphenylphosphorylazide) to give the amides of structure (63).

Treatment of Compound (59) with an isocyanate gives the ureas (61).

The pharmaceutically-acceptable salts of the present invention may be synthesized from the compounds of Formula I which contain a basic moiety by conventional chemical methods. Generally, the salts are prepared by reacting the free base with stoichiometric amounts of or with an excess of the desired salt-forming inorganic or organic acid in a suitable solvent or in various combinations of solvents.

Screening of the novel compounds according to the present invention to determine biological activity and obtain an IC₅₀ value for them, in order to identify significant CCK-antagonism, may be accomplished using an ¹²⁵I-CCK-receptor binding assay and in vitro isolated tissue preparations. In order to identify significant gastrin antagonism, ¹²⁵I-gastrin and ³H-pentagastrin binding assays may be used. These tests involve the following:

### CCK receptor binding (pancreas) method

CCK-8, radiolabeled with ¹²⁵I-Bolton Hunter reagent (2000 Ci/mmole) is purchased from New England Nuclear (NEN) and receptor binding is performed according to Innis and Snyder (Proc. Natl. Acad. Sci., 77, 6917-6921, 1980), with minor modifications as described in Chang and Lotti (Proc. Natl. Acad. Sci., 83, 4923-4926, 1986).

The whole pancreas of a male Sprague-Dawley rat (200-350 g), which has been sacrificed by decapitation, is dissected free of fat tissue and homogenized in 20 volumes of ice-cold 50 mM Tris HCl (pH 7.7 at 25°C) with a Brinkmann Polytron PT-10. The homogenates are centrifuged at 48,000 g for 10 minutes, then the resulting pellets are resuspended in Tris Buffer, centrifuged as above, and resuspended in 200 volumes of binding assay buffer (50 mM Tris HCl, pH 7.7 at 25°C, 5 mM dithiothreitol and 0.1 mM bacitracin).

For the binding assay, 25 ml of buffer (for total binding), or unlabeled CCK-8 sulfate sufficient to give a final concentration of 1 mM of CCK-8 (for nonspecific binding), or the compounds according to the instant invention (for determination of antagonism to ¹²⁵I-CCK binding) and 25 ml of ¹²⁵I-CCK-8 (30,000-40,000 cpm), are added to 450 ml of the membrane suspensions in duplicate or triplicate test tubes. The reaction mixtures are incubated at 37°C for 30 minutes and then filtered on glass fiber GF/B filters, which are then rapidly washed with 3 x 4 ml of ice cold Tris HCl containing 1 mg/ml BSA, and the filters are counted with a Beckman Gamma 5000. For Scatchard analysis to determine the mechanism of inhibition of ¹²⁵I-CCK binding by the most potent compounds (Ann. N.Y. Acad. Sci., 51, 660, 1949), ¹²⁵I-CCK-8 is progressively diluted with increasing concentrations of CCK-8.

### CCK receptor binding (brain) method

¹²⁵I-CCK-8 binding is performed similarily to the method described by Saito et al. (J. Neurochem., 37, 483-490, 1981), with modifications described by Chang and Lotti (Proc. Natl. Acad. Sci., 83, 4923-4926, 1986).

Male Hartley guinea pigs (300-500 g) are sacrificed by decapitation, and the brains are removed and placed in ice-cold 50 mM Tris HCl (Trizma-7.4) [pH 7.4 at 25°C]. The cerebral cortex is dissected and used as a receptor source and each gram of fresh guinea pig brain tissue is homogenized in 10 ml of Tris/Trizma buffer with a Brinkmann polytron PT-10. The homogenates are centrifuged at 42,000g for 15 minutes, then the resulting pellets are resuspended in 200 volumes of binding assay buffer (10 mM N-2-hydroxyethyl-piperazine-N′-2-ethanesulfonic acid (HEPES), 5 mM MgCl₂, 1 mM ethylene glycol-bis-(β-aminoethylether)-N,N′-tetraacetic acid (EGTA), 0.4% BSA (bovine serum albumin) and 0.25 mg/ml bacitracin, (pH 6.5).

The remainder of the binding assay method is as described for the pancreas method, except that the reaction mixtures are incubated at 25°C for 2 hours before centrifugation.

### Isolated guinea pig gall bladder method

The two halves of the gall bladders, free of adjacent tissue, of male Hartley guinea pigs (400-600g), which have been sacrificed by decapitation, are suspended under 1g tension along the axis of the bile duct in 5 ml organ bath, containing a Kreb'S bicarbonate solution of 118 mM NaCl, 4.75 mM KCl, 2.54 mM CaCl₂, 1.19 mM KH₂PO₄, 1.2 mM MgSO₄, 25 mM NaHCO₃ and 11 mM dextrose, which is maintained at 32°C and bubbled with a mixture of 95% 02 and 5% CO₂.
The tissues are washed every 10 minutes for one hour to obtain equilibrium prior to the beginning of the study and the isometric contractions of the strips are recorded using Statham (60g:0.12 mm) strain gauges and a Hewlett-Packard 77588 recorder.

CCK-8 is added cumulatively to the baths and EC₅₀'s are determined using regression analysis. After washout (every 10 minutes for one hour), the compound to be tested is added at least 5 minutes before the addition of CCK-8 and the EC₅₀ of CCK-8 in the presence of compound to be tested is similarly determined.

A shift to the right of the CCK dose response curve without reduction of the maximal centractile response, indicates competitive antagonism of CCK from this method.

### Isolated longitudinal muscle of guinea pig ileum

Longitudinal muscle strips with attached nerve plexus are prepared as described in Brit. J. Pharmac. 23: 356-363, 1964; J. Physiol. 194: 13-33, 1969. Male Hartley guinea pigs are decapitated and the ileum removed (10 cm of the terminal ileum is discarded and the adjacent 20 cm piece used), with a 10 cm piece of the ileum being stretched on a glass pipette. Using a cotton applicator to stroke tangentially away from the mesentery attachment at one end, the longitudinal muscle is separated from the underlying circular muscle and the longitudinal muscle is tied to a thread and by gently pulling, stripped away from the entire muscle. A piece of approximately 2 cm is suspended in 5 ml organ bath containing Krebs solution and bubbled with 95% O₂ and 5% CO₂ at 37°C under 0.5 g tension. CCK-8 is added cumulatively to the baths and EC₅₀ values in the presence and absence of compounds to be tested are determined, as described in the gall bladder protocol above.

### Gastrin Receptor Binding in Guinea Pig Gastric Glands

Guinea pig gastric mucosal glands are prepared by the procedure of Berglingh and Obrink, Acta Physiol. Scand. 96: 150 (1976), with a slight modification according to Praissman et al. C. J. Receptor Res. 3: (1983). Gastric mucosa from male Hartley guinea pigs ( 300-500 g body weight) are washed thoroughly and minced with fine scissors in standard buffer consisting of the following: 130 mM NaCl, 12 mM NaHCO₃, 3 mM NaH₂PO₄, 3 mM Na₂HPO₄, 3 mM K₂HPO₄, 2 mM MgSO₄, 1 mM CaCl₂, 5 mM glucose, 4 mM L-glutamine and 25 mM HEPES at pH 7.4. The minced tissues are washed and incubated in a 37°C shaker bath for 40 minutes, with the buffer containing 0.1% collagenase and 0.1% BSA, and bubbled with 95% O₂ and 5% CO₂. The tissues are passed twice through a 5 ml glass syringe to liberate the gastric glands, and then filtered through 200 mesh nylon. The filtered glands are centrifuged at 270 g for 5 minutes and washed twice by resuspension and centrifugation.

The washed guinea pig gastric glands are resuspended in 25 ml of standard buffer containing 0.25 mg/ml of bacitracin. For binding studies, 10 ml of buffer (for total binding) or gastrin (1 mM final concentration, for nonspecific binding) or test compound and 10 ml of ¹²⁵I-gastrin (NEN, 2200 Ci/mmole, 25 pM final) or ³H-pentagastrin (NEN, 22 Ci/mmole, 1 nM final) are added to 220 ml of gastric glands in triplicate tubes which are aerated with 95% O₂ and 5% CO₂, and capped. The reaction mixtures, after incubation at 25°C for 30 minutes, are filtered under reduced pressure on glass G/F B filters (Whatman) and immediately washed with 4 x 4 ml of standard buffer containing 0.1% BSA. The radioactivity on the filters is measured using a Beckman gamma 5500 for ¹²⁵I-gastrin or liquid scintillation counting for ³H-pentagastrin.

The pharmaceutical compositions containing the compounds of Formula I may be useful in the treatment or prevention of central nervous system disorders including neurological and pyschiatric disorders. Examples of such central nervous system disorders include anxiety disorders and panic disorders. Additional examples of central nervous system disorders include panic syndrome, anticipatory anxiety, phobic anxiety, panic anxiety, chronic anxiety, and endogenous anxiety.

The pharmaceutical compositions containing the compounds of Formula I may further be used to control pupil constriction in the eye. The compounds may be used for therapeutic purposes during eye examinations and intraocular surgery in order to prevent miosis. The compunds may further be used to inhibit miosis occurring in association with iritis, uveitis and trauma.

The pharmaceutical compositions containing the compounds of Formula I may further be used for preventing or treating the withdrawal response produced by chronic treatment or abuse of drugs or alcohol. Such drugs include, but are not limited to cocaine, alcohol or nicotine.

The compounds of the instant invention or pharmaceutically-acceptable salts thereof, may be administered to a human subject either alone or, preferably, in combination with pharmaceutically-acceptable carriers or diluents, optionally with known adjuvants, such as alum, in a pharmaceutical composition, according to standard pharmaceutical practice. The compounds can be administered orally or parenterally, including intravenous, intramuscular, intraperitoneal, subcutaneous and topical administration.

For oral use of an antagonist of CCK, according to this invention, the selected compounds may be administered, for example, in the form of tablets or capsules, or as an aqueous solution or suspension. In the case of tablets for oral use, carriers which are commonly used include lactose and corn starch, and lubricating agents, such as magnesium stearate, are commonly added. For oral administration in capsule form, useful diluents include lactose and dried corn starch. When aqueous suspensions are required for oral use, the active ingredient is combined with emulsifying and suspending agents. If desired, certain sweetening and/or flavoring agents may be added. For intramuscular, intraperitoneal, subcutaneous and intravenous use, sterile solutions of the active ingredient are usually prepared, and the pH of the solutions should be suitably adjusted and buffered. For intravenous use, the total concentration of solutes should be controlled in order to render the preparation isotonic.

When a compound according to Formula I is used as an antagonist of CCK or gastrin in a human subject, the daily dosage will normally be determined by the prescribing physician with the dosage generally varying according to the age, weight, and response of the individual patient, as well as the severity of the patient's symptoms. However, in most instances, an effective daily dosage will be in the range of from about 0.005 mg/kg to about 50 mg/kg of body weight, and preferably, of from about 0.05 mg/kg to about 50 mg/kg of body weight, and most preferably, of from about 0.5 mg/kg to about 20 mg/kg of body weight administered in single or divided doses.

In some cases, however, it may be necessary to use dosage levels outside these limits. For example, doses as low as about 1 ng/kg, about 0.005 µg to about 0.05 µg, or about 100 ng to about 100 µg/kg may be administered.

In the effective treatment of panic syndrome, panic disorder, anxiety disorder and the like, preferably about 0.05 mg/kg to about 0.5 mg/kg of CCK antagonist maybe administered orally (p.o.), administered in single or divided doses per day (b.i.d.). Other routes of administration are also suitable.

The invention is further defined by reference to the following examples which are intended to be illustrative and not limiting.

### EXAMPLE 1

Preparation of 6-phenyl-4H-pyrrolo[1,2-a][1,4]benzodiazepine (14, X¹=H, R²=Ph, R¹=R¹³=R¹⁷=H)

This compound is prepared according to the method of Hara et al, J. Med. Chem., 21, 263-268 (1978).

### EXAMPLE 2

Preparation of 4-oximino-6-phenyl-4H-pyrrolo [1,2-a]-1,4-benzodiazepine (58, X¹= H, R²= Ph, W = Y = Z = CH)

To a suspension of potassium tert-butoxide (24.9 g, 222 mmole) in 600 mL of dry tetrahydrofuran is added 200 mL of dry tert-butylalcohol at -20°C under nitrogen. To this solution is then added, via addition funnel, 6-phenyl-4H-pyrrolo[1,2-a]-1,4-benzodiazepine (25 g) in 260 mL of tetrahydrofuran. The resulting solution is stirred for about 2 hours at -20°C and treated with 17.4 mL (130 mmole) of isoamyl nitrite. The reaction mixture is warmed to 0°C over approximately 15 minutes and quenched with the addition of 60 mL of cold water and 20 mL of glacial acetic acid. All solvents are removed under reduced pressure and the residue is partitioned between ethyl acetate (600 mL) and brine (100 mL). The phases are separated and the organic extracts are dried (Na₂SO₄) and concentrated. The resulting product is triturated with ether to give Compound (58).

### EXAMPLE 3

Preparation of 4(R,S)-amino-6-phenyl-4H-pyrrolo [1,2-a]-1,4-benzodiazepine (59, X¹= H, R²= Ph, W = Y = Z = CH, n = O)

A solution of 150 mL of methanol containing 5 g 4-oximino-6-phenyl-4H-pyrrolo[1,2-a]-1,4-benzodiazepine is treated with a slurry of active Raney-nickel catalyst¹ in ethanol (10 g). The resulting suspension is hydrogenated on a Parr apparatus at 60 psi and 23°C for about 30 hour. The catalyst is removed by filtration and the filtrate is concentrated to afford the title compound.
¹The Raney-Nickel catalyst is prepared according to Fieser & Fieser, Reagents for Organic Synthesis, Vol. I, John Wiley & Sons, Inc., New York 1967, p. 729.

### EXAMPLE 4

Preparation of 4(R,S)-(2(S)-tert-butoxycarbonylamino-3-phenylpropanoylamino)-6-phenyl-4H-pyrrolo[1,2-a]-1,4-benzodiazepine

Crude 4(R,S)-amino-6-phenyl-4H-pyrrolo-[1,2-a]-1,4-benzodiazepine (1.37 g), Boc-L-phenylalanine (1.37 g, 5.17 mmole), 1-hydroxybenzotriazole (HBT) (0.70 g, 5.17 mmole), and 1-ethyl-3-(3-dimethylaminopropyl)carbodiimide hydrochloride (EDC) (0.99 g, 5.17 mmole) are combined in DMF (30 mL) and stirred at room temperature. The pH of the reaction mixture is adjusted to 8.5 with triethylamine. After approximately 1/2 hour, the DMF is removed in vacuo and the residue is partitioned between ethyl acetate and 10% citric acid solution (10 mL). The layers are separated and the organic phase is washed with sodium bicarbonate solution (NaHCO₃, saturated). The combined organic layers are washed with brine, dried over Na₂SO₄, filtered, and evaporated to dryness in vacuo. The residue is chromatographed on silica gel and the combined product fractions evaporated to dryness in vacuo to give the title compound as a mixture of diastereomers.

### EXAMPLE 5

Preparation of 4(R and S)-(2(S)-Amino-3-phenylpropanoylamino)-6-phenyl-4H-pyrrolo[1,2-a]-1,4-benzodiazepine

4(RS)-(2(S)-tert-Butoxycarbonylamino-3-phenylpropanoylamino)-6-phenyl-4H-pyrrolo[1,2-a]-1,4-benzodiazepine (1.8 gm) is dissolved in EtOAc (25 mL), cooled to 0°C, and the solution saturated with HCl (g) over a 10 minute period. After stirring an additional 10 minutes, the solvent is removed in vacuo. The residue is dissolved in H₂O, basified with saturated Na₂CO₃ (aqueous) and extracted with EtOAc (3x). The organic layers are combined, washed with brine, dried over Na₂SO₄, filtered and rotoevaporated in vacuo to give a foam. Flash chromatography on silica gel separates the 1/1 pair of diastereomers into an upper and a lower component. The individual fractions containing the components are concentrated to dryness to give the separated diastereomers.

### EXAMPLE 6

Preparation of 4(R)- and 4(S)-Amino-6-phenyl-4H-pyrrolo[1,2-a]-1,4-benzodiazepine

4(S)-(2(S)-amino-3-phenylpropanoylamino)-6-phenyl-4H-pyrrolo[1,2-a]-1,4-benzodiazepine (1.15 g) is combined with phenylisothiocyanate (395 mg, 2.93 mmole) in CH₂Cl₂ (20 mL) and the mixture concentrated on a steam bath. The resulting product is twice diluted with CH₂Cl₂ (20 mL) and both times reconcentrated on the steam bath. The product is evaporated in vacuo to a foam which is treated with TFA (15 mL) and warmed for 18 minutes in an oil bath thermostated at 52°, with the TFA being removed in vacuo. The residue is treated twice with CH₂Cl₂ and with Et₂O, evaporated in vacuo after each treatment, and the resulting product chromatographed on silica gel. The product fractions are evaporated in vacuo, and the residue is dissolved in CH₂Cl₂, washed with a small volume of 5% NaOH, dried over Na₂SO₄, filtered, and evaporated to give the 4-(S) isomer of the title structure.

4(R)-(2(S)-amino-3-phenylpropanoylamino)-6-phenyl-4H-pyrrolo[1,2-a]-1,4-benzodiazepine was converted by the same procedure to the 4-(R) enantiomer of the title compound.

### EXAMPLE 7

Preparation of 4(S)-4-(2-indolecarbonylamino)-6-phenyl-4H-pyrrolo-[1,2-a]-1,4-benzodiazepine (62, X¹=H R²=Ph, R³=NHCO-2-indole, W=Y=Z=H)

4(S)-4-Amino-6-phenyl-4H-pyrrolo[1,2-a]-1,4-benzodiazepine (595 mg) is dissolved in CH₂Cl₂ (15 mL) and treated with 2-indolecarbonyl chloride (403 mg, 2.24 mmole) followed by triethylamine (227 mg, 2.24 mmole). The mixture is stirred at room temperature for approximately 30 minutes and concentrated in vacuo. The residue is chromatographed on silica gel and the combined product fractions evaporated to dryness in vacuo. Three times, Et₂O (15 mL) is added and evaporated in vacuo to give the title compound.

### EXAMPLE 8

4(R)-4(3-Methoxyphenylaminocarbonylamino)-6-phenyl-4H-pyrrolo[1,2-a]-1,4-benzodiazepine (61, X¹= H, R²Ph, 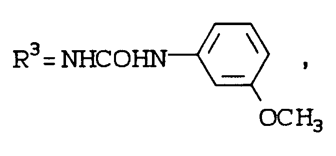
W=Y=Z=H)

To a solution of 85 mg of 4(R)-amino-6-phenyl-4H-pyrrolo[1,2-a]-1,4-benzodiazepine in 8 ml of dry tetrahydrofuran is stirred in 3-methoxyphenylisocyanate (40 ml, 0.315 mmole) at room temperature. Stirring is continued for 8 more hours and the reaction mixture is filtered with the collected product being washed with hot methanol and dried in vacuo.

### EXAMPLE 9

Preparation of 6-phenyl-4H-imidazo[1,2-a][1,4] benzodiazepine (1, Scheme III, X = H, R¹= H, R²= phenyl, R³= H)

This compound is prepared according to the method of Gall and Kamdar, J. Org. Chem., 46, 1575-1585 (1981).

### EXAMPLE 10

Preparation of 4-oximino-6-phenyl-4H-imidazo[1,2-a]-1,4-benzodiazepine(58, X¹= H, R²= Ph, W = Y = GH, Z = N)

This compound is prepared according to the method of Example 2.

### EXAMPLE 11

4-Amino-6-phenyl-4H-imidazo[1,2-a]-1,4-benzodiazepine (59, X¹= H, R²= Ph, W = Y = Ch, Z = N, n = O)

This compound is prepared according to the method of Example 3 and resolved according to the methods of Examples 4 through 6.

### EXAMPLE 12

4(S)-4-(4-Chlorophenylcarbonylamino)-6-phenyl-4H-imidazo[1,2-a]-1,4-benzodiazepine (1, X¹= H, R²=phenyl, R¹=H,

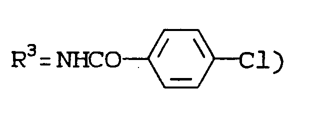

This compound is prepared according to the method of Example 7.

### EXAMPLE 13

4(R)-4-(3-Methylphenylaminocarbonylamino)-6-phenyl-4H-imidazo[1,2-a]-1,4-benzodiazepine (1, X¹= H, R¹= H, R²=phenyl,

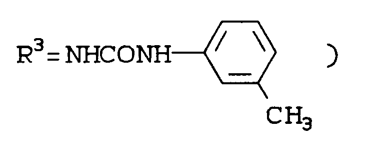

This compound is prepared according to the method of Example 8.

### EXAMPLE 14

1-Methyl-6-phenyl-3-propyl-4H-imidazo[1,5-a]-1,4-benzodiazepine (1, Scheme V, X¹= H, R²= Ph, R¹= methyl, R³= H, R¹⁷= propyl)

This compound is prepared according to the methods of Walser et al, J. Heterocyclic Chem., 15, 855-858 (1978).

### EXAMPLE 15

1-Methyl-4-oximino-6-phenyl-3-propyl-4H-imidazo-[1,5-a]-1,4-benzodiazepine (58, X¹= H, R²= Ph, W = C-CH₃, Y = N, Z = C-CH₂CH₂CH₃)

This compound is prepared according to the method of Example 2.

### EXAMPLE 16

4-Amino-1-methyl-6-phenyl-3-propyl-4H-imidazo[1,5-a]-1,4-benzodiazepine (59, X¹= H, R²= Ph, W = C-CH₃, Y = N, Z= C-CH₂CH₂CH₃, n = O)

This compound is prepared according to the method of Example 3 and resolved according to the methods of Example 4 through 6.

### EXAMPLE 17

4(S)-4-(2-Indolecarbonylamino)-1-methyl-6-phenyl-3-propyl-4H-imidazo[1,5-a]-1,4-benzodiazepine (1, X¹= H, R¹= methyl, R²= Ph, R³= NHCO-2-indole, R¹⁷= n-propyl)

This compound is prepared according to the method of Example 7.

### EXAMPLE 18

4(R)-4-(3-Chlorophenylaminocarbonylamino)-1-methyl-6-phenyl-3-propyl-4H-imidazo[1,2-a]-1,4-benzodiazepine (1, X¹= H, R¹= methyl, R²= Ph, R³= 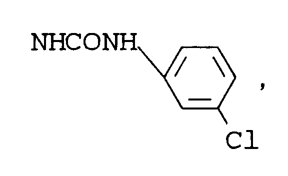
R¹⁷=n-propyl)

This compound is prepared according to the method of Example 8.

### EXAMPLE 19

6-Phenyl-4H-pyrazolo[1,5-a][1,4]benzodiazepine-2-carboxylic acid, ethyl ester (40, X¹= H, R²= Ph, R¹¹= CH₂CH₃)

This compound is prepared according to the methods of Gilman et al, J. Heterocyclic Chem., 14, 1163 (1977).

### EXAMPLE 20

4-Oximino-6-phenyl-4H-pyrazolo[1,5-a][1,4]benzodiazepine-2-carboxylic acid, ethyl ester (58, X¹= H, R²= Ph, W = N, Y = COOCH₂CH₃, Z = CH)

This compound is prepared according to the method of Example 2.

### EXAMPLE 21

4-Amino-6-phenyl-4H-pyrazolo[1,5-a][1,4]benzodiazepine-2-carboxylic acid, ethyl ester (59, X¹= H, R²= Ph, W = N, Y = COOCH₂CH₃, Z = CH)

This compound is prepared according to the method of Example 3 and resolved according to the methods of Examples 4 through 6.

### EXAMPLE 22

4(R)-4-(3-Methoxyphenylaminocarbonylamino)-6-phenyl-4H-pyrazolo[1,5-a][1,4]benzodiazepine-2-carboxylic acid, ethyl ester. (61, X = H, R²= Ph, R³= 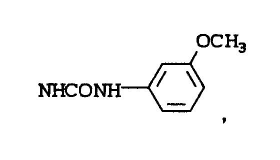
W=N, Y=C-COOCH₂CH₃, Z=CH)

This compound is prepared according to the method of Example 8.

### EXAMPLE 23

4-(S)-4-(2-Indolecarbonylamino)-2-methyl-6-phenyl-4H-pyrazolo [1,5-a][1,4]benzodiazepine (62, X¹= H, R²= Ph, R³= NHCO-2-indole, W = N, Y = C-CH₃, Z= CH)

This compound is prepared according to the methods of Examples 19 through 21 and 7.

### EXAMPLE 24

3-Methyl-6-phenyl-6H-triazolo[1,5-a][1,4]-benzodiazepine(48, X¹= H, R²= Ph)

This compound is prepared according to the methods of Coffen et al, J. Org. Chem., 40, 894 (1975).

### EXAMPLE 25

3-Methyl-4-Oximino-6-phenyl-4H-triazolo[1,5-a][1,4]-benzodiazepine (58, X¹=H, R²= Ph, W=Y=N, Z=C-CH₃)

This compound is prepared according to the method of Example 2.

### EXAMPLE 26

4-Amino-3-methyl-6-phenyl-4H-triazolo[1,5-a][1,4]-benzodiazepine (59, X¹= H, R²= Ph, W = Y = N, Z = C-CH₃)

This compound is prepared according to the method of Example 3 and resolved according to the methods of Examples 4 through 6.

### EXAMPLE 27

4(S)-4-(2-Indolecarbonylamino)-3-methyl-6-phenyl-4H-triazolo[1,5-a][1,4]-benzodiazepine (62, X¹= H, R²= Ph, R³= NHCO-2-indole, W = Y = N, Z = C-CH₃)

This compound is prepared according to the method of Example 7.

### EXAMPLE 28

4(R)-4-(3-Methylphenylaminocarbonylamino)-3-methyl-6-phenyl-4H-triazolo[1,5-a][1,4]-benzodiazepine (61, X¹=H, R²=Ph, 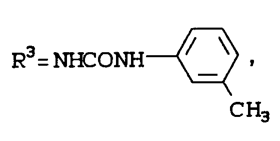
W=Y=N, Z = C-CH₃)

This compound is prepared according to the method of Example 8.

### EXAMPLE 29

6-Phenyl-4H-tetrazolo[1,5-a][1,4]benzodiazepine (1, Scheme VII, X = H, R²= Ph, R³= H)

This compound is prepared according to the methods of Hester, et al, Tetrahedron Lett., 20, 1609 (1971).

### EXAMPLE 30

4-Oximino-6-phenyl-4H-tetrazolo[1,5-a][1,4]benzodiazepine (58, X¹= H, R²= Ph, W = Y = Z = N)

This compound is prepared according to the method of Example 2.

### EXAMPLE 31

4-Amino-6-phenyl-4H-tetrazolo[1,5-a][1,4]benzodiazepine (59, X¹= H, R²= Ph, W = Y = Z = N)

This compound is prepared according to the method of Example 3 and resolved according to the methods of Examples 4 through 6.

### EXAMPLE 32

4(S)-4-(2-Indolecarbonylamino)-6-phenyl-4H-tetrazolo-[1,5-a][1,4]benzodiazepine (1, Scheme VIII, X¹= H, R²= Ph, R³= NHCO-2-indole)

This compound is prepared according to the method of Example 7.

### EXAMPLE 33

4(R)-4-(3-Chlorophenylaminocarbonylamino)-6-phenyl-4H-tetrazolo[1,5-a][1,4]benzodiazepine(1, Scheme VIII, X¹=H, R²=Ph,

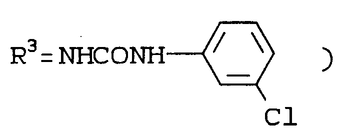

This compound is prepared according to the method of Example 8.

## Claims

1. A pharmaceutical composition useful in the treatment of panic disorder or anxiety disorder, comprising a therapeutically effective amount of a compound of the formula: 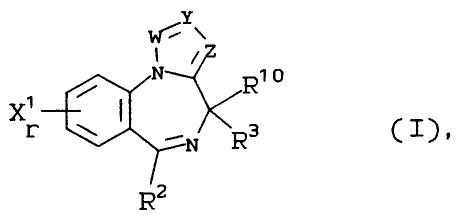 wherein
R¹ is H, C₁-C₄-straight- or branched-chain alkyl, cyclo-C₃-C₇-alkyl, or unsubstituted or mono- or disubstituted phenyl, where the substituent(s) is/are selected from the group consisting of halo, C₁-C₄-alkyl, C₁-C₄-alkoxy, and hydroxy;
R² is H, C₁-C₄-straight- or branched-chain alkyl, mono- or disubstituted or unsubstituted phenyl, where the substituent(s) is/are selected from the group consisting of halo, C₁-C₄-alkyl, C₁-C₄-alkoxy, C₁-C₄-alkylthio, carboxyl, carboxyl-C₁-C₄-alkyl, nitro, -CF₃, 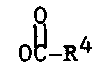 and hydroxy, 2-, 3- or 4-pyridyl, or -(CH₂)ₘCOOR⁶;
R³ is 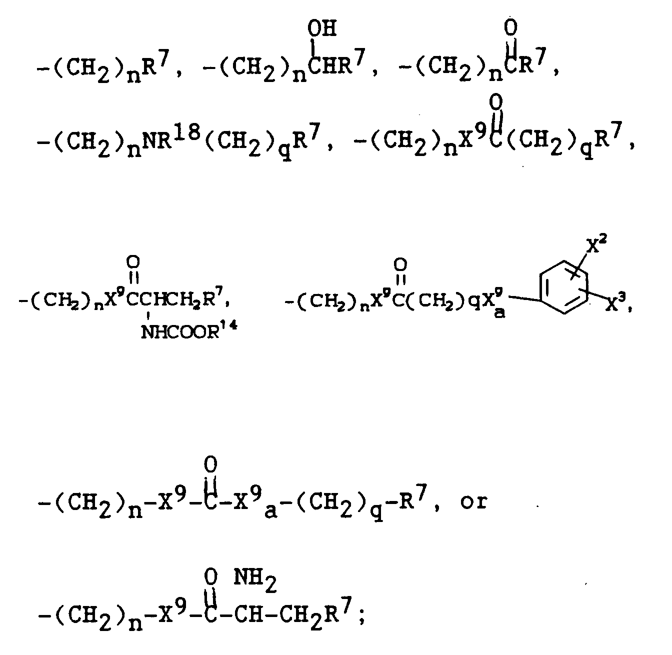 R⁴ and R⁵ are independently H, C₁-C₄-straight- or branched-chain-alkyl, cyclo-C₃-C₇-alkyl , or are connected to form a hetero ring of the form, -N(CH₂)ₖ, where k is 2 to 6;
R⁶ is H, C₁-C₄-straight or branched-chain alkyl, cyclo-C₃-C₇-alkyl, unsubstituted or mono- or disubstituted phenyl (where the substituent(s) is/are selected from the group consisting of halo, C₁-C₄-alkyl, C₁-C₄-alkoxy, nitro, and CF₃), or unsubstituted or mono- or disubstituted phenyl-C₁-C₄-straight or branched-chain alkyl (where the substituent(s) is/are selected from the group consisting of halo, C₁-C₄-alkyl, C₁-C₄-alkoxy, nitro, and CF₃);
R⁷ is a- or β-naphthyl, unsubstituted or mono- or disubstituted phenyl (where the substituent(s) is/are selected from the group consisting of halo, -NO₂, -OH, -NR⁴R⁵, C₁-C₄-straight- or branched-chain alkyl, cyano, phenyl, trifluoromethyl, acetylamino, acetyloxy, C₁-C₄-straight- or branched-chain alkylthio, SCF₃, C=CH, CH₂SCF₃, OCHF₂, S-phenyl, or C₁-C₄-straight- or branched-chain alkoxy), 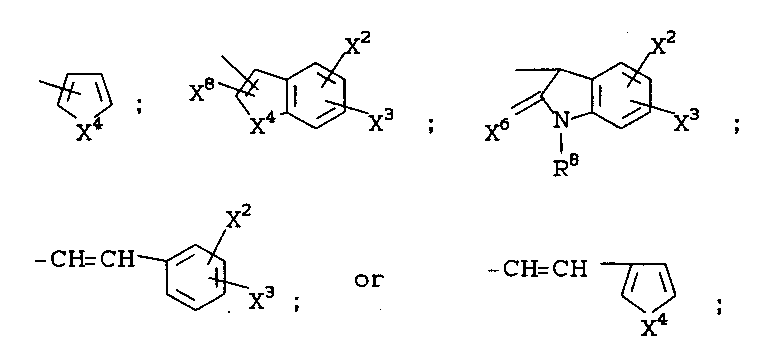 R⁸ is H, C₁-C₄-straight- or branched-chain alkyl, cyclo-C₃-C₇-alkyl, -(CH₂)ₙ-cyclo-C₃-C₇-straight- or branched-chain alkyl, 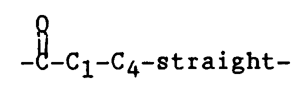 or branched-chain alkyl, or 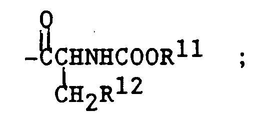 R⁹ is OH, OR¹¹ or 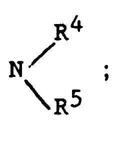 R¹⁰ is H, -OH, or -CH₃;
R¹¹ and R¹² are independently C₁-C₄-straight- or branched-chain alkyl or cyclo-C₃-C₇-alkyl;
R¹³ is H or C₁-C₄-straight- or branched-chain alkyl, cyclo-C₃-C₇-alkyl, 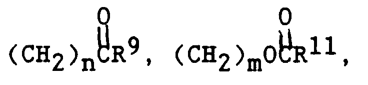 (CH₂)ₘNHR¹⁵, CH₂)ₘOH, or phenyl-C₁-C₄-straight- or branched-chain alkyl;
R¹⁴ is C₁-C₄-straight- or branched-chain alkyl or phenyl-C₁-C₄-straight- or branched-chain alkyl;
R¹⁵ is H or 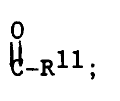 R¹⁶ is H, C₁-C₄-straight- or branched-chain alkyl, cyclo-C₃-C₇-alkyl, or phenyl-C₁-C₄-straight- or branched-chain alkyl;
R¹⁷ is H, C₁-C₄-straight- or branched-chain alkyl, COOR¹⁶, phenyl or phenyl-C₁-C₄-straight- or branched-chain alkyl;
R¹⁸ is H, C₁-C₄-straight- or branched-chain alkyl or formyl, acetyl, propionyl or butyryl;
m is 1-to-4;
n is 0-to-4;
q is 0-to-4;
r is 1 or 2;
X¹ is H, -NO₂, CF₃, CN, OH, C₁-C₄-straight- or branched-chain alkyl, halo, C₁-C₄-straight- or branched-chain alkylthio, C₁-C₄-straight- or branched-chain alkoxy, -(CH₂)ₙCOOR⁶, -NR⁴R⁵, or 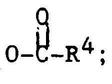 X² and X³ are independently H, -OH,-NO₂, halo, C₁-C₄-straight- or branched-chain alkylthio, C₁-C₄-straight- or branched-chain alkyl, C₁-C₄-straight- or branched-chain alkoxy, or 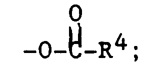 X⁴ is S, O, CH₂, or NR⁸;
X⁶ is O or HH;
X⁸ is H or C₁-C₄-straight- or branched-chain alkyl;
X⁹ and X⁹ₐ are independently NR¹⁸ or O;
W = CR¹ or N;
Y = CR¹³ or N;
Z = N or CR¹⁷;
or a pharmaceutically acceptable salt thereof and a pharmaceutically acceptable carrier.

2. A pharamceutical composition useful in the treatment of panic disorder or anxiety disorder, comprising a therapeutically effective amount of a compound of Claim 1 wherein:
R¹ is H or C₁-C₄-alkyl; R² is unsubstituted or mono- or disubstituted phenyl, wherein the substituents are selected from the group consisting of halo, C₁-C₄-alkyl, C₁-C₄-alkoxy, nitro and -CF₃; R³ is -(CH₂)ₙR⁷, 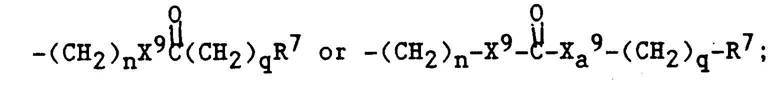 R⁴ and R⁵ are independently H, C₁-C₄-alkyl or are connected to form the ring -N(CH₂)ₖ, where k is 4 or 5; R⁷ is a- or β-naphthyl, unsubstituted or mono- or disubstituted phenyl, wherein the substituents are selected from the group consisting of halo, -NO₂, C₁-C₄-alkyl, C₁-C₄-alkoxy and trifluoromethyl, 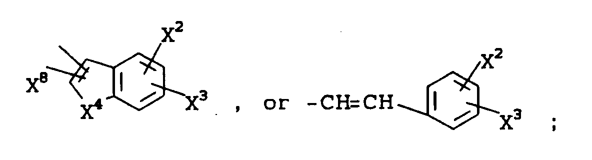 R⁸ is H or C₁-C₄-alkyl; R¹¹ is C₁-C₄-alkyl; R¹³ is H, C₁-C₄-alkyl, cyclo-C₃-C₅-alkyl or 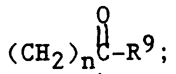 R¹⁶ is C₁-C₄-alkyl; n is 0-to-2; q is 0-to-2; X¹ is H, -NO₂, -C₁-C₄-alkyl, halo or C₁-C₄-alkoxy; X² and X³ are independently H, -NO₂, halo, C₁-C₄-alkyl or C₁-C₄-alkoxy; and X⁹ and Xₐ⁹ are independently NH or O; or a pharmaceutically acceptable salt thereof and a pharmaceutically acceptable carrier.

3. A pharmaceutical composition useful in treatment of panic disorder of anxiety disorder, comprising a therapeutically effective amount of a compound of Claim 2, wherein:
R¹ is H or methyl; R² is phenyl or o-F-phenyl; R³ is 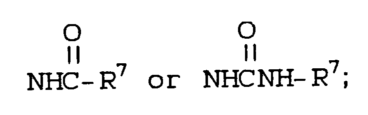 R⁷ is 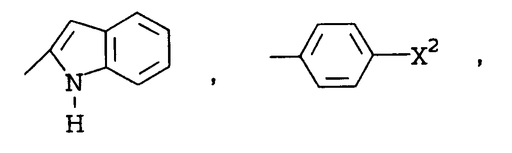 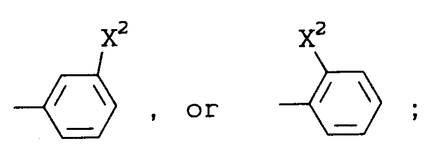 R¹³ is H, methyl, ethyl or propyl; R¹⁷ is H, methyl or ethyl; X¹ is H; X² is H, -NO₂, halo, methyl or methoxy; W is CR¹; Y is CR¹³ and Z is CR¹⁷; or a pharmaceutically acceptable salt thereof and a pharmaceutically acceptable carrier.

4. A pharmaceutical composition useful in the treatment of panic disorder or anxiety disorder, comprising a therapeutically effective amount of a compound of Claim 2, wherein
R¹ is H or methyl; R² is phenyl or o-F-phenyl; R³ is 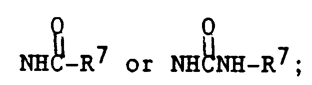 R⁷ is 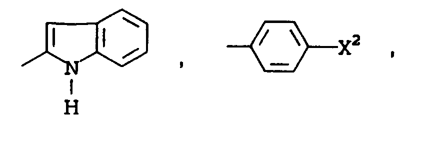 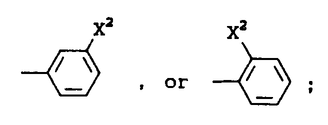 R¹³ is H; X¹ is H; X² is H, -NO₂, halo, methyl or methoxy; W is CR¹; Y is CR¹³; and Z is N; or a pharmaceutically acceptable salt thereof and a pharmaceutically acceptable carrier

5. A pharmaceutical composition useful in the treatment of panic disorder or anxiety disorder, comprising a therapeutically effective amount of a compound of Claim 2, wherein:
R¹ is H or methyl; R² is phenyl or o-F-phenyl; R³ is 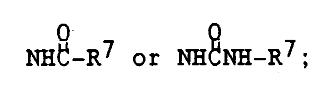 R⁷ is 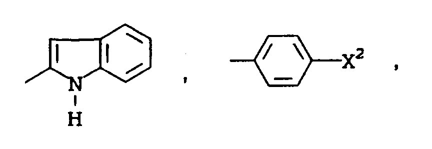 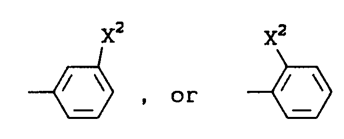 R¹⁷ is H, CH₃, CH₂CH₃, COOH, COOCH₃, or COOCH₂CH₃; X¹ is H; X² is H, -NO₂, halo, methyl, or methoxy; W is CR¹; Y is N and Z is CR¹⁷; or a pharmaceutically acceptable salt thereof and a pharmaceutically acceptable carrier.

6. A pharmaceutical composition useful in the treatment of panic disorder or anxiety disorder, comprising a therapeutically effective amount of a compound of Claim 2 wherein:
R² is phenyl or o-F-phenyl; R³ is 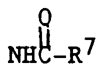 or 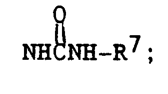 R⁴ and R⁵ are independently H, methyl, ethyl or -(CH₂)₄-; R⁷ is 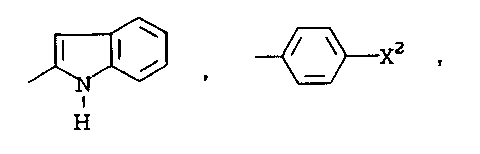 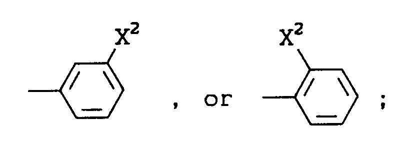 R⁹ is OH, or OCH₃; R¹³ is 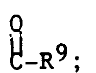 R¹⁷ is H; X¹ is H; X² is H, -NO₂, halo, methyl, or methoxy; W is N; Y is CR¹³; and Z is CR¹⁷; or a pharmaceutically acceptable salt thereof and a pharmaceutically acceptable carrier.

7. A pharmaceutical composition useful in the treatment of panic disorder or anxiety disorder, comprising a therapeutically effective amount of a compound of Claim 2, wherein:
R² is phenyl or o-F-phenyl; R³ is 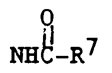 or 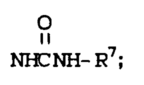 R⁷ is 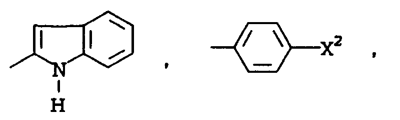 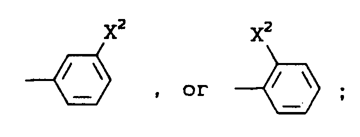 R¹⁷ is H, methyl, COOCH₃, or COOCH₂CH₃; X¹ is H; X² is H, -NO₂, halo, methyl or methoxy; W is N; Y is N; and Z is CR¹⁷; or a pharmaceutically acceptable salt thereof and a pharmaceutically acceptable carrier.

8. A pharmaceutical composition useful in the treatment of panic disorder or anxiety disorder, comprising a therapeutically effective amount of a compound of Claim 2, wherein:
R² is phenyl or o-F-phenyl; R³ is 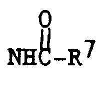 or 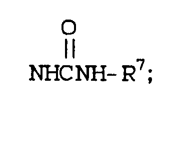 R⁷ is 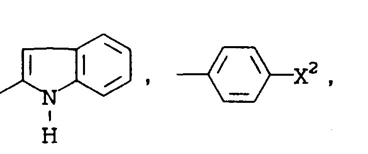 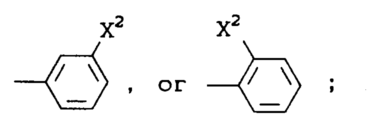 X¹ is H; X² is H, -NO₂, halo, methyl or methoxy; W is N; Y is N; and Z is N; or a pharmaceutically acceptable salt thereof and a pharmaceutically acceptable carrier.

9. A pharmaceutical composition useful in the treatment of panic disorder or anxiety disorder, comprising a therapeutically effective amount of a compound of Claim 2, which is:
4(S)-4(2-indolecarbonylamino)-6-phenyl-4H-pyrrolo-[1,2-a]-1,4-benzodiazepine;
4(S)-4(4-chlorophenylcarbonylamino)-6-phenyl-4H-imidazo-[1,2-a]-1,4-benzodiazepine;
4(S)-4-(2-indolecarbonylamino)-1-methyl-6-phenyl-3-propyl-4H-imidazo-[1,5-a]-1,4-benzodiazepine;
4(S)-4-(2-indolecarbonylamino)-2-methyl-6-phenyl-4H-pyrazolo[1,5-a]-1,4-benzodiazepine;
4(S)-4-(2-indolecarbonylamino)-3-methyl-6-phenyl-4H-triazolo-[1,5-a]-1,4-benzodiazepine; or
4(S)-4-(2-imdolecarbonylamino)-6-phenyl-4H-tetrazolo-[,5-a]-1,4-benzodiazepine.

10. A pharmaceutical composition useful in the treatment of panic disorder or anxiety disorder, comprising a therapeutically effective amount of a compound of Claim 2, which is:
4(R)-4(3-methoxyphenylaminocarbonylamino)-6-phenyl-4H-pyrrolo[1,2-a]-1,4-benzodiazepine;
4(R)-4-(3-methylphenylaminocarbonylamino)-6-phenyl-4H-imidazo-[1,2,-a]-1,4-benzodiazepine;
4(R)-4-(3-chlorophenylaminocarbonylamino)-1-methyl-6-phenyl-3-propyl-4H-imidazo-[1,2-a]-1,4-benzodiazepine;
4(R)-4-(3-methoxyphenylaminocarbonylamino)-6-phenyl-4H-pyrazolo-[1,5-a]-1,4-benzodiazepine-2-carboxylic acid, ethyl ester;
4(R)-4-(3-methylphenylaminocarbonylamino)-3-methyl-6-phenyl--4H-triazolo-[1,5-a]-1,4-benzodiazepine; or
4(R)-4-(3-chlorophenylaminocarbonylamino)-6-phenyl-4H-tetrazolo-[1,5-a]-1,4-benzodiazepine.

11. A pharmaceutical composition useful for controlling pupil constriction in the eye, or treating a withdrawal response produced by chronic treatment or abuse or drugs or alcohol, comprising a therapeutically effective amount of a compound of Claim 1, or a pharmaceutically acceptable salt thereof and a pharmaceutically acceptable carrier.

12. A pharmaceutical composition according to Claim 1 or Claim 2, wherein the therapeutically effective amount of the compound of Formula I is from about 0.005 mg/kg to about 50 mg/kg of body weight, administered in a single or divided dose.

13. The use of a compound of Claim 1 for the manufacture of a medicament for treating panic disorder or anxiety disorder in a mammal.

14. The use of a compound according to Claim 1 for the manufacture of a medicament for controlling pupil constriction in the eye, or treating a withdrawal response produced by chronic treatment or abuse of drugs or alcohol.

15. The use as claimed in Claim 13 or Claim 14, wherein the compound of Formula I is present in the medicament manufactured in an amount of from about 0.005 mg/kg to about 50 mg/kg of body weight, administered in a single or divided dose.
